(19) Europäisches Patentamt
European Patent Office
Office européen des brevets

(11) Publication number: **0 236 307 B1**

# (12) EUROPEAN PATENT SPECIFICATION published in accordance with Art. 158(3) EPC

(45) Date of publication of patent specification: **17.04.91**

(51) Int. Cl.[5]: **C07K 5/06**, //A61K37/02

(21) Application number: **85904731.8**

(22) Date of filing: **16.09.85**

(86) International application number: **PCT/US85/01744**

(87) International publication number: **WO 87/01707 (26.03.87 87/07)**

## (54) ANTIHYPERTENSIVE AGENTS, PHARMACEUTICAL COMPOSITIONS CONTAINING THEM AND PROCESSES FOR THE PREPARATION OF THE AGENTS AND COMPOSITIONS.

(43) Date of publication of application: **16.09.87 Bulletin 87/38**

(45) Publication of the grant of the patent: **17.04.91 Bulletin 91/16**

(84) Designated Contracting States: **AT BE CH DE FR GB IT LI LU NL SE**

(56) References cited:
EP-A- 0 088 350
EP-A- 0 121 830
EP-A- 0 126 986
EP-A- 0 127 124
EP-A- 0 135 182
US-A- 4 468 396

(73) Proprietor: **SCHERING CORPORATION**
**2000 Galloping Hill Road**
**Kenilworth New Jersey 07033(US)**

(72) Inventor: **DOLL, Ronald, James**
**126 Union Avenue**
**Maplewood, NJ 07040(US)**
Inventor: **NEUSTADT, Bernard, Ray**
**24 Brook Place**
**West Orange, NJ 07052(US)**
Inventor: **SMITH, Elizabeth, Melva**
**166 Grove Avenue**
**Verona, NJ 07044(US)**
Inventor: **MAGATTI, Charles, Victor**
**63 Cumberland Avenue**
**Verona, NJ 07044(US)**
Inventor: **GOLD, Elijah, Herman**
**10 Roosevelt Avenue**
**West Orange, NJ 07052(US)**

(74) Representative: **von Kreisler, Alek, Dipl.-Chem. et al**
**Deichmannhaus am Hauptbahnhof**
**W-5000 Köln 1(DE)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention relates to (benzothiadiazine, benzamido, and benzenesulfonyl)-phenyl-substituted carboxyalkyl dipeptide compounds which have antihypertensive activity. Compounds of this invention are useful as antihypertensive agents, in the treatment of congestive heart failure and glaucoma. In addition, compounds of this invention are useful as diuretics.

US-A-4,468,396 deals with benzothiadiazinyl-substituted carboxyalkyl dipeptides useful as combined antihypertensive and diuretic agents.

More particularly, this invention relates to compounds represented by the following formula:

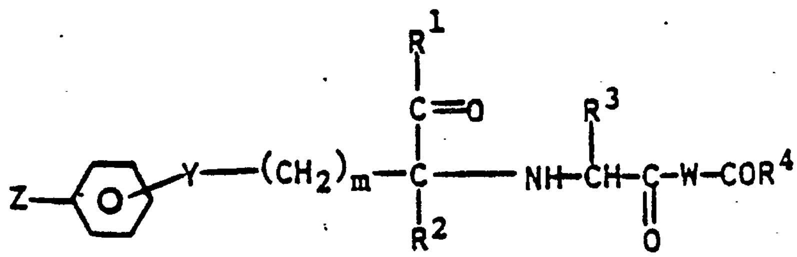

I

wherein W is

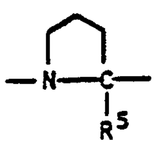
,
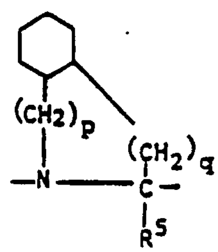
,
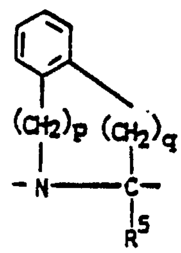
,
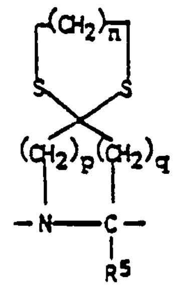
;

II III IV V

n is 0 or 1; m is 0 to 2

p and q are each 0, 1 or 2, provided that the sum of p and q is 1 or 2, and that in formula V, p is not 0;

Y is $-CH_2-$, $-CH_2O-$, or $-CR_2S-$, attached at the 2 or 4 position of the phenyl group;

Z is

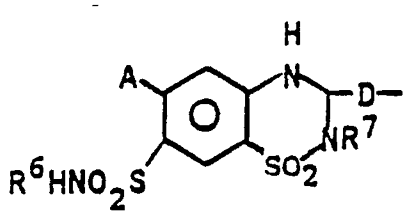
,
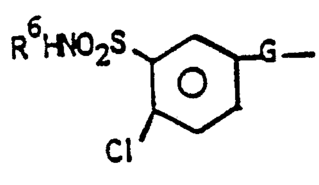

VI VII

wherein A is Cl or $CF_3$;

D is $-(CH_2)_u-$, $-CH_2O-$,$-CH_2S-$;

$$-CH_2\overset{\overset{O}{\|}}{C}NH-;$$

G is $-CONR^7(CH_2)_t-$, or $-SO_2NR^7(CH_2)t-$; t is 0 or 1;

$R^1$ and $R^4$ are independently hydroxy, alkoxy having from 1 to 8 carbon atoms, $K-X_r-(CH_2)_s-O-$, wherein

K is phenyl, substituted phenyl, 1- or 2-naphthyl, X is oxygen or sulfur, r is 0 or 1 and s is 0 or 4, and wherein the substitutents on the phenyl are chosen from group M, wherein M is halogen, hydroxy, trifluoromethyl, alkoxy having from 1 to 6 carbon atoms, alkyl from 1 to 6 carbon atoms, 2- and 3-furanyl, 2- and 3-thienyl and phenyl (which phenyl group may be substituted with halogen, hydroxy, trifluoromethyl, alkoxy having from 1 to 6 carbon atoms or alkyl having from 1 to 6 carbon atoms), provided that when s is zero, r is zero, $-OCH_2-OCO$-alkyl wherein the alkyl has from 3 to 8 carbon atoms, $-OCH_2CO$- phenyl, wherein the phenyl may be substituted with group M, 1-glyceryl,

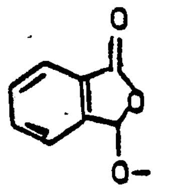
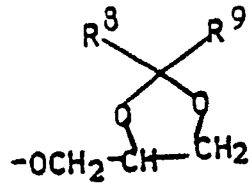

$R^2$, $R^5$, $R^6$ and $R^9$ are hydrogen or $C_1$-$C_6$ alkyl;
$R^3$ is hydrogen, $C_1$-$C_6$ alkyl or amino lower alkyl;
$R^7$ is hydrogen, $C_1$-$C_6$ alkyl or phenyl($C_1$-$C_6$)alkyl;
$R^8$ is hydrogen, $C_1$-$C_6$ alkyl, phenyl, or phenyl substituted by group M;
u is 1 or 2;
and the pharmaceutically acceptable salts thereof.

Preferred compounds of the invention are those wherein W is represented by formula III, IV or V. When W is of formula III or IV, preferred values for p and q are 0 and 1, respectively; when W is of formula V, preferred values of p, q and n are 1, 1 and 0 respectively.

Two additional groups of preferred compounds are that wherein $R^2$ and $R^5$ are hydrogen, and that wherein $R^4$ is hydroxy.

Particularly preferred compounds are those wherein W is represented by formula III or V; n, p, q, Y, $R^2$, and $R^5$ are as defined above for preferred compounds; $R^3$ is methyl or amino butyl; Z is of formula VI, wherein A is chlorine, or Z is of formula VII and G is $-CONH-CH_2$-or $-SO_2NH-CH_2$; $R^6$ and $R^7$ are hydrogen or methyl; and $R^1$ is hydroxy, ethoxy, methoxy, phenoxyethoxy, or pivaloyloxymethoxy.

As used herein, "$C_1$-$C_6$ alkyl" means straight or branched chain hydrocarbon radicals of from 1 to 6 carbons, e.g. methyl, ethyl, propyl, ispropyl, butyl, t-butyl, pentyl and hexyl. Similarly, "$C_1$-$C_6$ alkoxy" means straight or branched alkoxy radicals having 1 to 6 carbon atoms, e.g. methoxy, ethoxy, propoxy, butoxy, iso-butoxy, pentoxy and hexyloxy. "Halogen" means fluorine, chlorine and bromine.

Compounds of the instant invention include various stereoisomers. Preferred stereoisomers are those in which the absolute configuration at each of the three carbon atoms adjacent to both a nitrogen and a carbonyl group corresponds most closely to the absolute configuration of L-amino acids.

The compounds of this invention form salts with various inorganic and organic acids and bases which are also within the scope of the invention. Such salts include ammonium salts, alkali metal salts, e.g. sodium and potassium salts, and alkaline earth metal salts, e.g. calcium and magnesium salts. Salts with organic and inorganic acids may be prepared, e.g., HCl, HBr, $H_2SO_4$, $H_3PO_4$, methanesulfonic acid, toluenesulfonic acid, maleic acid, fumaric acid and camphorsulfonic acid. The non-toxic pharmaceutically acceptable salts are preferred, although other salts are also useful, e.g., in isolating or purifying the product. The acid salts (e.g. HCl and maleate) are preferred, especially the hydrochloride.

The salts may be formed by conventional means, as by reacting the free acid or base forms of the product with one or more equivalents of the appropriate base or acid in a solvent or medium in which the salt is insoluble, or in a solvent such as water which is then removed in vacuo or by exchanging the cations of an existing salt for another cation on a suitable ion exchange resin.

Compounds of formula I may be prepared by several routes using methods known in the art.

For example, compounds of formula I may be prepared by condensing an amino acid of formula VIII with a keto compound of formula IX in the presence of a reducing agent such as sodium cyanoborohydride in a solvent such as ethanol:

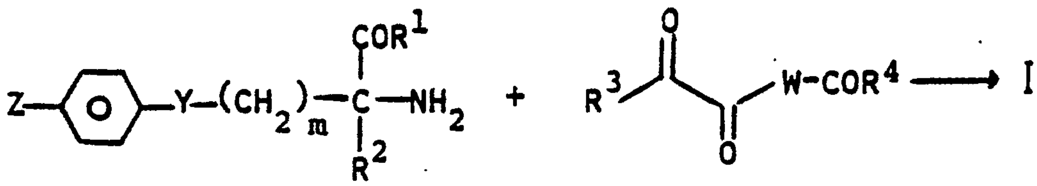

VIII IX

wherein Z, Y, $R^1$, $R^2$, $R^3$, $R^4$, m and W are as defined above.

Starting materials of formula VIII may be prepared by well known methods. An example of such a preparation is shown below, wherein 5-(4-[6-chloro-7-sulfamoyl-3,4-dihydro-1,1-dioxo-1,2,4-benzothiadiazinyl-3-)methoxy]benzyl)cysteine (formula XVI) is prepared, starting with 2-bromo-1,1-diethoxyethane and p-cresol:

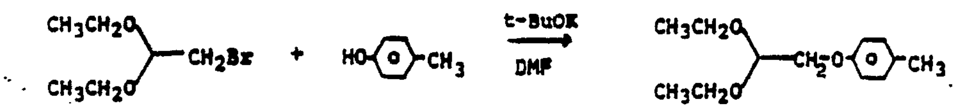


X XI XII

XII

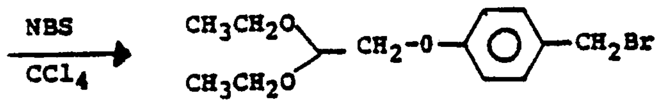


XIII

XIII +

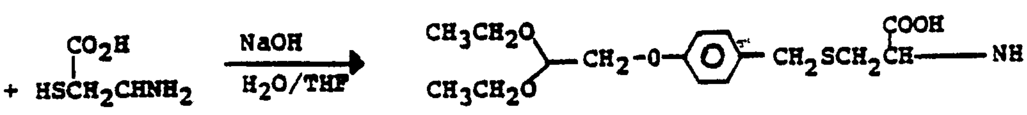


XIV

XIV

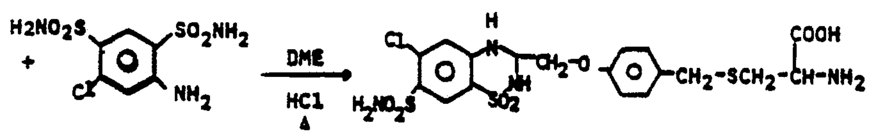


XV XVI

Starting materials of formula IX may be prepared by reacting an amino acid derivative XVIII with an α-keto acid chloride XVII to give the substituted amino acid:

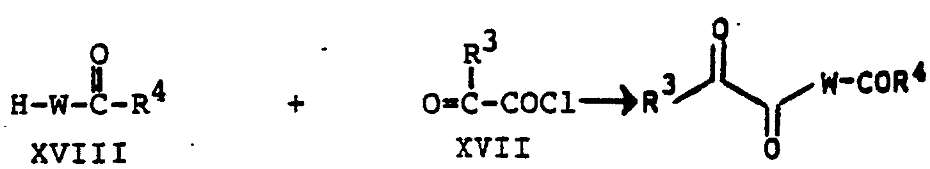

XVIII XVII

IX

The reaction is carried out in an inert solvent such as methylene chloride in the presence of a base such as triethylamine or pyridine.

Compounds of formula I wherein Y is $-CH_2-$, and Z is a group of formula VII, are preferably prepared by the reaction of an acid of formula XIX with an amine of formula XX:

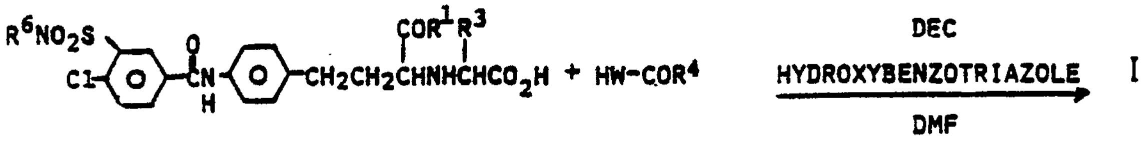


XIX XX I

wherein $R^1$, $R^3$, $R^4$, $R^6$ and W are as defined above, and "DEC" refers to 1-(3-dimethylaminopropyl)-3-ethyl carbodiimide HCl.

Compounds of formula XIX may be prepared from known starting materials by techniques well known in the art. The following reaction scheme describes a method of preparing a compound of formula XIX (designated formula XIXa):

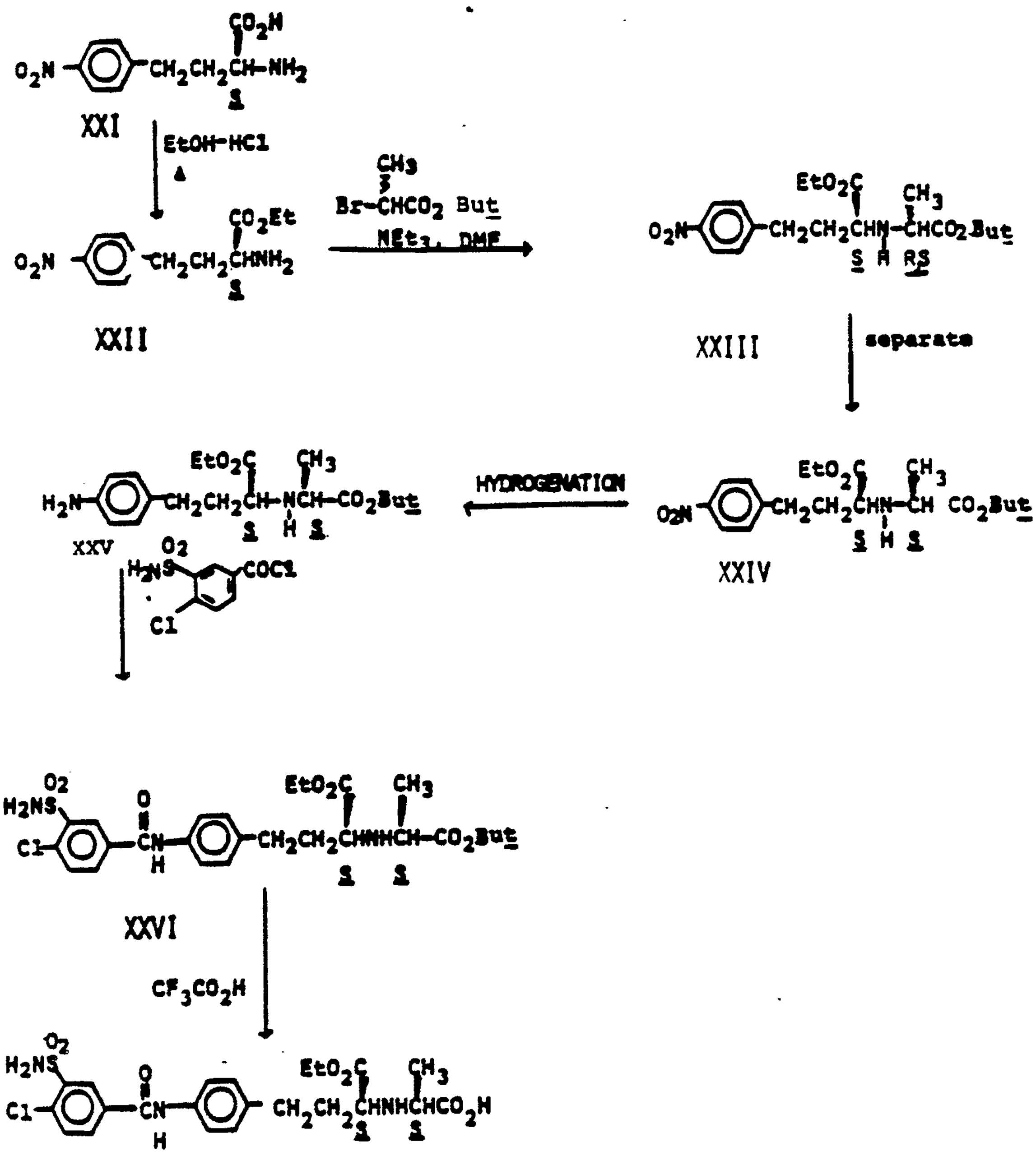


XXI XXII XXIII XXIV XXV XXVI XIXa

A preferred method for the conversion of compounds of formula XXII to XXIV in the reaction scheme

above which eliminates the preparation of diastereomers of formula XXIII and their subsequent separation is to use the specific diastereomer t-butyl 2R-(trifluoromethanesulfonyloxy)propionate (triflate reagent). In this process, the single diastereomer of the triflate reagent reacts by nucleophillic displacement with the α-aminoacid ester (e.g., a compound of formula XXII) to give a high yield of the corresponding specific single diastereomer of the resulting monoamino dicarboxylic acid ester (e.g., a compound of formula XXIV).

Since the preferred compounds of formula I have an S-configuration at the carbon to which $R^3$ is attached the triflate reagent used herein is the 2-R diastereomer (see Preparation 4). However, the process is generally applicable to converting a broad range of α-aminoacid esters to the desired specific single diastereomer by using the appropriate triflate diastereomer. In place of the t-butyl ester of the triflate, other lower alkyl esters or the benzyl ester may be used.

The reaction proceeds at room temperature (i.e., 20-50 °C, preferably about 25 °C) in an inert solvent such as chloroform, dichloromethane, carbontetrachloride, benzene, toluene, or ethyl acetate in the presence of a base such as a tertiary amine (e.g., triethylamine or N-methylmorpholine). The reaction is complete in about 24 hours or less. The desired compound is recovered from the reaction mixture and purified by standard techniques. For example, the crude product is extracted into an organic solvent such as ether and concentrated to a crude oil, which is then purified by column chromatography to yield the desired specific diastereomer.

Carboxy-protected compounds of formula XX are prepared by methods well known in the art. See, for example, Neustadt et al. in European Patent Application 50,800, published May 5, 1982.

Alternatively, compounds of formula I wherein Y is $-CH_2-$, and Z is a group of formula VII may be prepared by the reaction of an acid chloride of formula XXVII with a dipeptide of formula XXVIII:

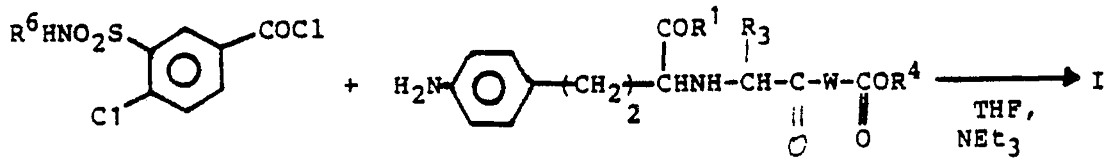


XXVII XXVIII

wherein $R^1$, $R^3$, $R^4$, $R^6$ and W are as defined above.

Compounds of formula XXVII may be prepared by known methods.

Compounds of formula XXVIII may be prepared by well known methods, an example of which is shown in the following reaction scheme:

XXIV

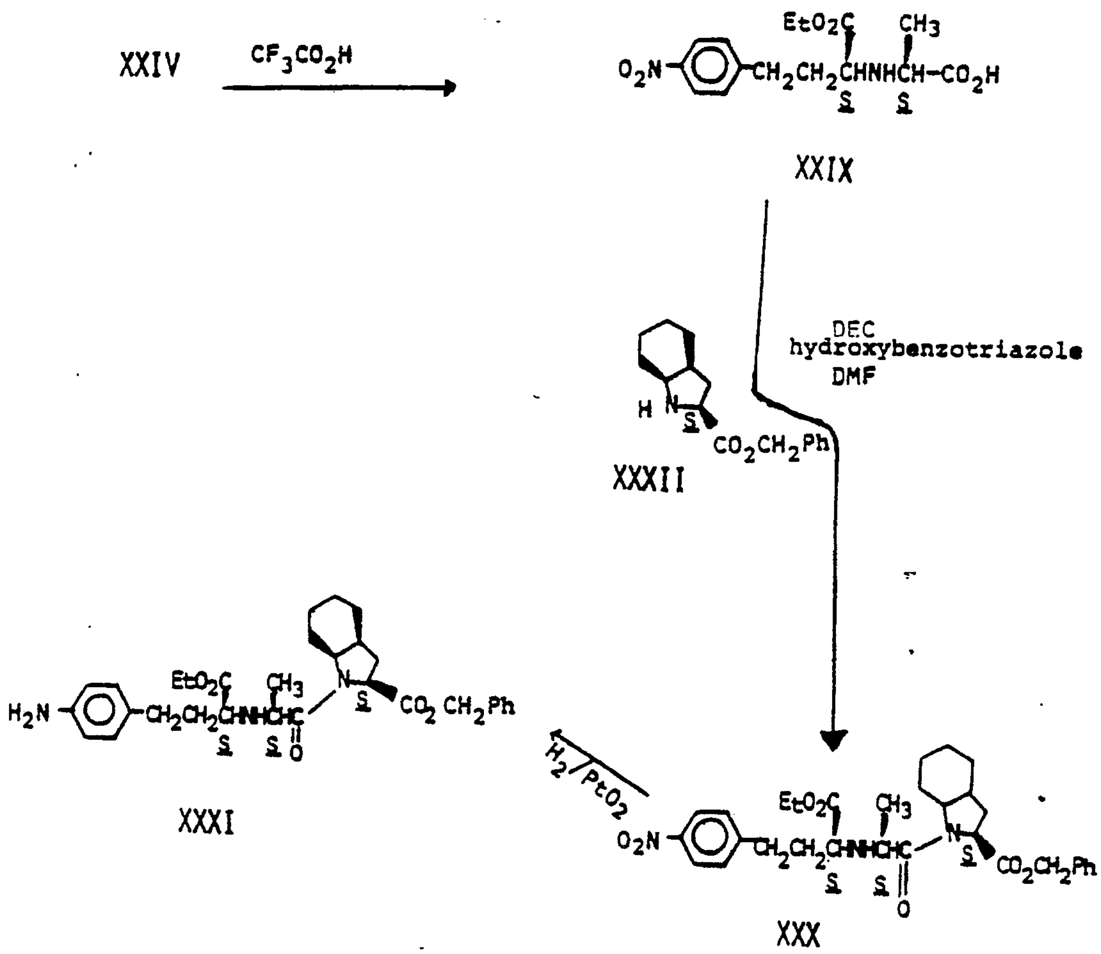


XXIX

XXXII

XXXI

XXX

XXXI

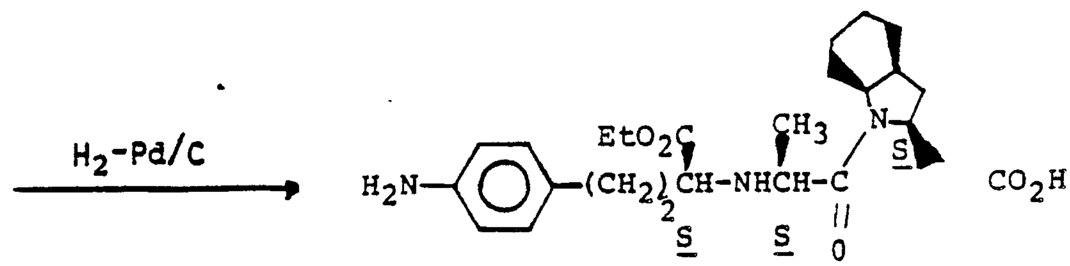


XXVIIIa

Alternatively, XXX may be hydrogenated directly to give XXVIIIa using a catalyst such as palladium on carbon.

Alternatively, XXXII may be reacted with a compound of formula XIXa to give a compound of formula I wherein $R^4$ is benzyloxy. The benzyloxy group may be then removed by hydrogenation with an appropriate catalyst such as palladium on carbon.

The known coupling methods above include amino group protection during the coupling reaction, for example by N-formyl, N-t-butoxycarbonyl and N-carbobenzyloxy groups, followed by their removal to yield compounds of formula I. Furthermore, the $COR^4$ function wherein $R^4$ is OH may be protected by removable ester groups such as benzyl, ethyl, t-butyl and the like.

If desired the so obtained compounds can be subjected to salt formation and/or esterification and/or trans-esterification and/or de-esterification according to known methods. If necessary the individual isomers can be be isolated according to known methods.

The more complex esters at $R^1$ (i.e., $R^1$ is other than hydroxy or alkoxy) are most conveniently

prepared by esterifying compounds of formula I wherein $R^1$ is hydroxy and $R^4$ is benzyloxy with the appropriate reagent, then removing the benzyl ester at $R^4$. For example, compounds of formula I where $R^1$ is hydroxy and $R^4$ is benzyloxy may be reacted with chloromethyl pivalate to obtain the corresponding pivaloyloxymethyl ester.

The following examples further illustrate the preparation of compounds of this invention.

PREPARATION 1

1-Pyruvoyl-cis,syn-Octahydro-1H-Indole-2(S)-Carboxylic Acid

A. Dissolve 27.0 g of ethyl indole-2-carboxylate in 250 ml of trifluoroacetic acid. Add 2.05 g of platinium oxide, hydrogenate the mixture at 50 lb/in$^2$ at room temperature. Filter the mixture and concentrate the filtrate *in vacuo* to give a residue. Suspend the residue in ether and treat with cold dilute sodium hydroxide solution. Dry the organic layer over magnesium sulfate and concentrate it to give ethyl octahydroindole-2-carboxylate, a pale yellow oil.

B. Dissolve 116 g of 10-d-camphorsulfonic acid in 1 liter of warm ethyl acetate and add a solution of 86 g of the product of part A in 1 liter of ethyl acetate. Allow the mixture to crystallize, heat to reflux, cool to room temperature, and filter. Recrystallize the filter cake from a mixture of 500 ml of isopropanol and 1800 ml ethyl acetate, filter and dry the crystals to obtain 2(S)-carboethoxy-*cis* ,*syn* -octahydro-1*H* -indole, d-10-camphorsulfonate, m.p. 192-193° C.

C. Slurry 10 g of the product of part B in 1 liter of ether, adjust to pH 11 with aqueous sodium hydroxide, and stir for 5 minutes. Wash the organic layer with sodium chloride solution, dry over magnesium sulfate, filter, and evaporate *in vacuo* at room temperature to obtain 2(S)-carboethoxy-*cis* ,*syn* -octahydro-1*H* -indole as a colorless oil. Dissolve the resultant oil in 50 ml of methanol containing 23 ml of 1*N* sodium hydroxide, stir at 25° C for 30 minutes, adjust to pH 7 with 1*N* hydrochloric acid, and evaporate the solvent to give *cis* ,*syn* -octahydro-1*H* -indole-2(S)-carboxylic acid.

D. Cool 23 ml of benzyl alcohol to 0° C under nitrogen and add 5.95 g of thionyl chloride dropwise over 15 minutes, maintaining the temperature at 0° C. Add the product of part C, stir for 1 hour at 0° C, then stir for 24 hours at room temperature. Pour the resulting mixture into 500 ml of ether, stir 1 hour under nitrogen, then allow to stand under nitrogen until the solution is clear. Decant the supernatant, wash the precipitate with 25 ml of ether, then slurry the precipitate in 200 ml of ether and adjust to pH 8-9 with 1-*N* sodium hydroxide. Stir 5 minutes, wash the organic layer with sodium chloride solution, dry over magnesium sulfate, filter and evaporate in vacuo at room temperature to obtain *cis* ,*syn* -octahydroindole-2(S)-carboxylic acid, benzyl ester as a colorless oil (TLC in ether: one spot, Rf 0.3).

E. To 26 g of the product of part D in 100 ml of dichloromethane and 7.8 ml of pyridine add 11.0 g of pyruvoyl chloride and stir the resulting mixture at room temperature. Extract the reaction mixture with water and dry the organic layer over magnesium sulfate. Concentrate the dichloromethane solution *in vacuo* and distill the residue to give 1-pyruvoyl-*cis* ,*syn* -octahydro-1*H* -indole-2(S)-carboxylic acid, benzyl ester.

F. To 20 g of the product from part E in 400 ml of ethanol, add 2.0 g of 10% palladium-on-charcoal and hydrogenate at 50 psi at room temperature. Filter the resulting mixture and concentrate the filtrate *in vacuo* to give the title compound.

PREPARATION 2

1-{N-[1(S)-Ethoxycarbonyl-2-(4-aminophenyl)ethyl]-(S)-alanyl}-cis,syn-octahydro-1H-indole-2(S)-carboxylic acid

Method I

A. To a solution of 4-nitrophenylalanine, ethyl ester, hydrochloride (54.0 g) in dry dimethylformamide (400 ml), add t-butyl 2-bromopropionate (112.3 g) and triethylamine (76 ml) and heat the resulting mixture at 70° for 18 hours under a nitrogen atmosphere. Pour the reaction mixture into water and extract with methylene chloride (6 x 300 ml). Combine the organic layers, dry over magnesium sulfate

and concentrate in vacuo to give a liquid (contains DMF). Chromatograph this liquid on a Prep 500 (3 silica gel cartridges) using hexane (8 l) then hexane:ethylacetate 4:1 and isolate N-[1(S)-ethoxycarbonyl-2-(4-nitrophenyl)ethyl]-(R)alanine, t-butyl ester, $[\alpha]_D^{26}$ = +24.7° (methanol), and N-1(S)-ethoxycarbonyl-2-(4-nitrophenyl)ethyl]-(S)alanine, t-butyl ester.

B. Add cold trifluoroacetic acid (600 ml) (ice bath) to N-[1(S)-ethoxycarbonyl-2-(4-nitrophenyl)ethyl]-(S)-alanine, t-butyl ester (25.5 g) and stir the resulting mixture at room temperature under a nitrogen atmosphere for 4 hours. Concentrate the solution in vacuo to give a viscous oil. Triturate the viscous oil with hexane (3 l) and then ether to yield N-[1(S)-ethoxycarbonyl-2-(4-nitrophenyl)ethyl]-(S)-alanine.

C. To a solution of the product of Step B (17.84 g), cis ,syn -octahydro-1H-indole-2(S)-carboxylic acid, benzyl ester (11.50 g), and triethylamine (4.46 g) in dimethylformamide (450 ml) at 0-5° under a nitrogen atmosphere, add 1-hydroxybenzotriazole (6.76 g) and 1-(3-dimethylaminopropyl)-3-ethylcarbodiimide, hydrochloride (16.13 g). Stir the reaction mixture at 0-5° for 25 minutes and then at room temperature for 90 minutes. Concentrate the reaction mixture in vacuo and partition between dichloromethane and saturated sodium bicarbonate solution. Dry the organic layer over magnesium sulfate and concentrate in vacuo to give a viscous oil which contains 1-{N-[1(S)-ethoxycarbonyl-2-(4-nitrophenyl)ethyl]-(S)-alanyl}-cis ,syn -octahydro-1H -indole-2(S)-carboxylic acid, benzyl ester.

D. Hydrogenate the product from Step C above in absolute ethanol (250 ml) in the presence of 10% palladium on carbon at 60 psi in a Parr Shaker Apparatus. Remove the catalyst by filtration through celite and concentrate the filtrate in vacuo to give a foam. Chromatograph the foam on the Prep 500 (3 cartriges) using chloroform:methanol:ammonium hydroxide 200:30:5 as eluant to give the title compound $[\alpha]_D^{26}$ = -44.0° (MeOH).

Method II

A. To a solution of 4-nitrophenylalanine, ethylester, hydrochloride (2.3 g) in dichloromethane (10 ml), add triethylamine (2.55 ml) and then t-butyl 2(R)-(trifluoromethanesulfonyloxy)propionate (2.80 g) (see Preparation 4) in dichloromethane (10 ml). Stir the resulting solution at room temperature for 20 hours. Concentrate the reaction mixture, add diethyl ether and extract with salt solution. Dry over magnesium sulfate and concentrate the ether solution in vacuo to give an oil. Place the oil on a column of silica gel (100 ml, 60-200 mesh) and elute with diethyl ether:hexane 60:40 to give N [1(S)-ethoxycarbonyl)-2-(4-nitrophenyl)ethyl]-(S)-alanine, t-butyl ester. B. to D. Proceed as described in Method I.

PREPARATION 3

1-{N[1(S)-Ethoxycarbonyl-3-(4-aminophenyl)propyl]-(S)-alanyl}-cis,syn-octahydro-1H-indole-2(S)-carboxylic acid

Method I

A. To a solution of 2-acetamido-4-(4-nitrophenyl)-butyric acid (57.65 g) in hot 95% ethanol (1000 ml) add d-(+)-α-methylbenzylamine (25.2 g) in hot 95% ethanol (125 ml), cool the solution slowly and keep at room temperature 18 hours. Collect the solid and wash with cold 95% ethanol, and dry to give an orange-yellow solid. Recrystallize this solid from 95% ethanol treated with charcoal to give 2(S)-acetamido-4-(4-nitrophenyl)-butyric acid, d-(+)-α-methylbenzyl amine salt $[\alpha]_D^{26}$ = +45.6 (MeOH), m.p. 211-213° C.

B. Suspend the product of part A (29.00 g) in ether (500 ml) and add 1N NaoH (150 ml). Separate the aqueous solution and wash with ether. Cool the aqueous solution in an ice-NaCl bath, add concentrated hydrochloric acid to pH 1 and stir the resulting mixture for 1 hour. Remove 2(S)-acetamido-4-(4-nitrophenyl)butyric acid, a white solid, $[\alpha]_D^{26}$ = + 33.9° (MeOH), m.p. 266° C.

C. Treat the compound prepared in part B above (18.65 g) with 6N hydrochloric acid (700 ml) and heat the resulting mixture under reflux for 2.5 hours. Concentrate the solution in vacuo to give 2(S)-amino-4-(4-nitrophenyl)butyric acid, hydrochloride, a solid, m.p. 186-189° C, $[\alpha]_D^{26}$ = +46.9° (MeOH).

D. Heat the compound prepared in part C (19.30 g) in absolute ethanol saturated with hydrogen chloride acid (400 ml) under reflux for 1-1/2 hour. Remove the solvent in vacuo and triturate the residue with ether to give 2(S)-amino-4-(4-nitrophenyl)butyric acid, ethyl ester, hydrochloride, a white solid m.p. 288.5° $[\alpha]_D^{26}$ + 40.6° (MeOH).

E. Treat the compound prepared in part D (18.00 g) in dry dimethylformamide (250 ml) with t-butyl 2-bromopropionate (35.20 g) and triethylamine (18.90 g) as described in Preparation 2A. Use Prep 500 (2 cartridges) and hexane (6 l) and then hexane/ethyl acetate 8:1 as eluants and isolate N-[1(S)-ethoxycarbonyl-3-(4-nitrophenyl)propyl]-(S)-alanine, t-butyl ester $[\alpha]_D^{26}$ = -8.0° (MeOH).

F. To the product of part E (6.90 g) at 0°, add trifluoroacetic acid (500 g) and treat the resulting mixture as described in Preparation 2B and isolate N-[1(S)-ethoxycarbonyl-3-(4-nitrophenyl)propyl]-(S)-alanine, trifluoroacetic acid salt, a viscous oil.

G. To a cold (0-5°) solution of the product of part F (6.68 g) and cis ,syn -octahydro-1H -indole-2(S)-carboxylic acid, benzyl ester (3.95 g) in anhydrous dimethylformamide (250 ml) and triethylamine (3.38 g), add 1-hydroxybenzotriazole (2.80 g) and 1-(3-dimethylaminopropyl)-3-ethylcarbodiimide, hydrochloride (5.85 g) and stir the resulting mixture at 0-5° C for 30 minutes and then at room temperature for 1.5 hour. Pour the reaction mixture into saturated sodium bicarbonate and extract with dichloromethane (2 x 1 l). Dry the organic layer over magnesium sulfate and concentrate in vacuo to give a viscous oil. Chromatograph this oil on the Prep 500 (2 cartridges) using ethyl acetate:hexane 3:20 and then 1:1 and isolate 1-{N[1(S)-ethoxycarbonyl-3-(4-nitrophenyl)propyl]-(S)-alanyl}-cis ,syn -octahydro-1H-indole-2(S)-carboxylic acid, benzyl ester.

H. Hydrogenate the product of part G (4.69 g) in absolute ethanol (250 ml) in the presence of 5% palladium-on-charcoal (0.50 g) at 60 psi in a Parr Shaker Apparatus. Remove catalyst by filtration and concentrate the filtrate to give the title compound, a foam $[\alpha]_D^{26}$ = -29.7° (MeOH).

Method II

A. to D. Proceed as described in Method I.

E. Treat the product of part D as described in Preparation 2, Method II, part A to obtain N-[1(S)-ethoxycarbonyl)-3-(4-nitrophenyl)propyl]-(S)-alanine, t-butyl ester.

F. to H. Proceed as described in Method I.

PREPARATION 4

t-Butyl 2R-(Trifluoromethanesulfonyloxy)Propionate

A. Add 2S -(p -toluenesulfonyloxy)propionic acid (4.4 g) to a cold solution of 10 ml isobutylene and 0.4 ml concentrated sulfuric acid in 30 ml methylene chloride in a pressure vessel, seal, and agitate at room temperaure for 48 hours. Pour into 50 ml 15% sodium carbonate solution, dry over magnesium sulfate and concentrate to obtain t-butyl 2S-(p-toluenesulfonyloxy)propionate as an oil (NMR 1.37). Distilled material (Kugelrohr, 120°) has $[\alpha]_D^{26}$ = -45.9° (EtOH, c=1).

B. Combine the product of part A (100 g) with acetic acid (40.0 g) and triethylamine (67.2 g) in 200 ml dry DMF. Heat at 65° for 20 hours. Partition with 2 l each ether and water, and wash the ether with citric acid, then with sodium bicarbonate solution. Dry and concentrate the ether solution to obtain t-butyl 2R -acetoxypropionate as a colorless liquid, bp 50° C/0.1 mm.

C. Combine the product of part B (62.6 g) with ethylenediamine (11.6 g) and heat at 70° for 24 hours. Allow to cool, add 300 ml ether and filter. Wash the ether with water, 10% citric acid, and then in sodium bicarbonate solution. Dry and concentrate the ether solution to leave a colorless oil. Crystallize from hexane at -20° to give t-butyl 2R -hydroxypropionate as white needles, m.p. 41-2° C.

D. Combine the product of part C (7.3 g) with pyridine (4.0 g) in 50 ml methylene chloride. Cool to -5° C, and add dropwise a solution of trifluoromethanesulfonic anhydride (14.1 g) in 25 ml methylene chloride. Allow the reaction to reach room temperature, then wash successively with water, 1N sulfuric acid and 1N sodium bicarbonate solution. Dry and concentrate the methylene chloride solution to leave the title compound as a colorless oil.

NMR (in CDC13) = 5.10 q; 1.73 d; 1.50 s.

EXAMPLE 1

1-{N-[1(S)-Ethoxycarbonyl-2-[4-(3-sulfamoyl-4-chlorobenzamido)phenyl]ethyl]-(S)-alanyl}-cis,syn-octahydro-1H-indole-2(S)-carboxylic acid

To a 0-5° C solution of the product of Preparation 2 (2.00 g) in anhydrous tetrahydrofuran (100 ml) and triethylamine (0.94 g), add a solution of 4-chloro-3-sulfamoylbenzoylchloride (1.61 g) in anhydrous tetrahydrofuran (10 ml) over a period of 30 minutes. Stir the resulting mixture for 15 minutes at 0-5° and then at room temperature for 18 hours. Filter the reaction mixture and concentrate the filtrate in vacuo to give a residue. Chromatograph the residue on the Prep 500 (1 cartridge) using chloroform:methanol: ammonium hydroxide 200:30:5 as eluant to give the title compound, a foam, $[\alpha]_D^{26}$ -16.1° (MeOH).

In a similar manner, using appropriate starting materials, prepare the following:

1-{N-[1(S)-ethoxycarbonyl-2-[4-(4-chloro-3-N-methyl-sulfamoylbenzamido)phenyl]ethyl]-(S)-alanyl}-cis ,syn -octahydro-1H -indole-2(S)-carboxylic acid, $[\alpha]_D^{26}$ = -18.7° (methanol).

1-{N-[1(S)-ethoxycarbonyl-3-[4-(2-hydroxy-4-chloro-5-sulfamoylbenzamido)phenyl]ethyl]-(S)-alanyl}-cis ,syn -octahydro-1H -indole-2(S)-carboxylic acid, $[\alpha]_D^{26}$ =-18.1° (methanol).

## EXAMPLE 2

### 1-{N-[1(S)-Carboxy-2-[4-(4-chloro-3-sulfamoylbenzamido)phenyl]ethyl]-(S)-alanyl}-cis,syn-octahydro-1H-indole-2(S)-carboxylic acid

To the product of Example 1 (0.35 g) add 0.5N NaOH (5 ml) and stir at room temperature for 1 hour. Add Bio-Rad Resin (AG 50W-X3, 100-200 mesh, hydrogen form) and then add to a column of the same resin. Elute with water (200 ml) and then water:pyridine 96:4. Concentrate the desired fractions to give the title compound. $[\alpha]_D^{26}$ = -7.0° (MeOH).

In a similar manner, prepare 1-{N[1(S)-carboxyl-2-[4-(4-chloro-3-N-methylsulfamoylbenzamido)phenyl]-ethyl]-(S)-alanyl}-cis,syn-octahydro-1H-indole-2(S)-carboxylic acid, $[\alpha]_D^{26}$ = 8.9° (methanol).

## EXAMPLE 3

### 1-{N-[1(S)-Ethoxycarbonyl-3-[4-(4-chloro-3-sulfamoylbenzamido)phenyl]propyl]-(S)-alanyl}-cis,syn-octahydro-1H-indole-2(S)-carboxylic acid

To a 0-5° solution of the compound of Preparation 3 (1.50 g) in anhydrous tetrahydrofuran (100 ml) and triethylamine (0.68 g), add a solution of 4-chloro-3-sulfamoylbenzoylchloride (1.11 g) and treat as described in Example 1, except use chloroform (2 l) and then chloroform:methanol:ammonium hydroxide100:30:5 as eluants and isolate the title compound, a foam $[\alpha]_D^{26}$ = -9.1 (methanol).

In a similar manner using appropriate starting materials, prepare 1-{N-[1(S)-Ethoxycarbonyl-3-[4-(4-chloro-3-N-methylsulfamoylbenzamido)phenyl]propyl]-(S)-alanyl}cis ,syn -octahydro-1H -indole-2(S)-carboxylic acid.

## EXAMPLE 4

### 1-{N-[1(S)-Carboxy-2-([4-[(6-Chloro-3,4-Dihydro-7-Sulfamyl-2H-1,2,4-Benzothiadiazin-3-yl-1,1-Dioxide)-Methyloxy]Phenyl]Methylthioethyl)-(S)-Alanyl}-cis,syn-Octahydro-1H-Indole-2(S)-Carboxylic Acid

A. Combine bromoacetaldehyde diethylacetal (19.7 g) and p-cresol (10.8 g) in dry dimethylformamide (DMF) (100 ml) and stir. Add potassium t-butoxide (9.6 g) and continue stirring for 24 hours, then evaporate the DMF in vacuo. Partition the resultant residue between ethyl acetate and water. Separate the organic layer, wash with 10% aqueous sodium hydroxide followed by brine, then dry the organic layer over sodium sulfate and filter. Evaporate the solvent in vacuo and purify the crude product on a silica gel column to obtain 4-[2,2-diethoxy)ethoxy]toluene:

NMR $\delta$ = 1.12 (6H, t,$CH_3$); 2.15 (s, 3H,-$CH_3$); 3.55 (q 4H, $CH_2$-0); 3.90(d, 2H, $CH_2$.phenyl); 4.77 (t,1H , -$CH_2$-); and 6.80 (m, 4H, Ar).

B. Combine N-bromosuccinamide (0.877 g) and the product of Step A (1 g) in carbon tetrachloride (20 ml) and stir at reflux for 18 hours. Filter the resultant solid and evaporate the solvent in vacuo to obtain 4-[(2,2-diethoxy)ethoxy]benzyl bromide:
NMR = 1.10 (t, 6H, CH); 3.59 (q, 4H, $-OCH_2$); 3.86 (d, 2H, $C-CH_2O$); 4.31(s, 2H, $CH_2$-Br); 4.70 (t, 1H , -CH-); 7.00 (m, 4H, Ar).

C. Combine methyl alcohol (20 ml) and 19 M sodium hydroxide (10 ml). Add L-cysteine (0.1 g), stir for 15 minutes, then add the product of Step B and stir at room temperature overnight. Adjust the resultant solution to approx. pH 7 and filter the resultant solid. Wash the solid with ether and dry under vacuum to obtain (S)-[4-[(2,2-diethoxy)ethoxybenzyl]cysteine.

D. Dissolve 4-amino-6-chloro-1,3-benzene-disulfonamide (0.74 g) in dimethoxyethane (10 ml), add the product of Step C (0.99 g), stir while heating to reflux, and add 2 drops of concentrated hydrochloric acid. Reflux 4 hours, then evaporate the solvent *in vacuo* . Wash the resultant solid with ether and dry under vacuum to obtain S-[4-[(6-chloro-3,4-dihydro-7-sulfamyl-2H-1,2,4-benzothiadiazin-3-yl-1,1-dioxide)-methoxy]benzyl-L-cysteine.

E. React 0.02 moles of the product of part D in 20 ml of tetrahydrofuran with 0.02 moles of the product of Preparation 1 and add 20 ml of molecular sieves 4A (Rohm and Haas). Stir the resulting mixture for 4 hours, add 12 g of sodium cyanoborohydride in 20 ml of methanol and stir the reaction mixture 20 hours. Filter, concentrate to dryness, and partition the residue between water and dichloromethane. Absorb the aqueous phase on strong acidic ion-exchange resin and elute with 4% pyridine in water. Separate the isomers on a column of silica gel using $CHCl_3$: isopropanol: 7% ammonium hydroxide 1:1:1 (organic phase) as eluant to give the title compound.

## EXAMPLE 5

1-{N-[1(S)-Ethoxycarbonyl-2-[4-(6-chloro-3,4-dihydro-1,1-dioxo-7-sulfamoyl-1,2,4-benzothiadiazine-3-acetamido)phenyl]ethyl]-(S)-alanyl}-cis,syn-octahydro-1H-indole-2(S)-carboxylic acid

A. Hydrogenate a solution of N-[1(S)-ethoxycarbonyl-2-(4-nitrophenyl)ethyl]-(S)-alanine, t-butyl ester (20.0 g) (see Preparation 2, IA) in absolute ethanol (500 ml) in the presence of 10% palladium on carbon (1.5 g) at 50 psi in a Parr shaker apparatus. Remove the catalyst by filtration and concentrate the filtrate *in vacuo* to give N-[1(S)-ethoxycarbonyl-2-(4-aminophenyl)ethyl]-(S)-alanine, t-butyl ester.

B. To a solution of the product of part A in dimethylformamide (150 ml), add 6-chloro-3,4-dihydro-1,1-dioxo-/-sulfamoyl-1,2,4-benzothiadiazine-3-acetic acid (14.4 g), 1-hydroxybenzotriazole (6.8 g) and 1-(3-dimethylaminopropyl)-3-ethylcarbodiimide, hydrochloride (9.6 g) at 0-5°. Warm the reaction mixture to room temperature and stir for 18 hours. Concentrate the reaction mixture *in vacuo* , add dichloromethane and concentrate *in vacuo* . Dissolve the residue in ethyl acetate and extract with 1N sodium bicarbonate. Dry (MgSO4) and concentrate the ethyl acetate solution *in vacuo* . Chromatograph the residue on silica gel using the Waters Prep 500 using ethylacetate as eluant to give N-[1(S)-ethoxycarbonyl-2-[4-(6-chloro-3,4-dihydro-1,1-dioxo-7-sulfamoyl-1,2,4-benzothiadiazine-3-acetamido)phenyl]ethyl]-(S)-alanine, t-butyl ester.

C. Treat the product (11.0 g) prepared in Example 5B with dioxane saturated with hydrogen chloride (100 ml) for 20 hours at RT. Concentrate the reaction mixture *in vacuo* and tritrate the residue with anhydrous ether to isolate N-[1(S)-ethoxycarbonyl-2-[4-(6-chloro-3,4-dihydro-1,1-dioxo-7-sulfamoyl-1,2,4-benzothiadiazine-3-acetamido)phenyl]ethyl-(S)-alanine hydrochloride salt.

D. Treat the product of part C as described in Preparation 2I, C to obtain {1-N-[1(S)-[ethoxycarbonyl-2-[4-(6-chloro-3,4-dihydro-1,1-dioxo-7-sulfamoyl-1,2,4-benzothiadiazine-3-acetamido)phenyl]ethyl]-(S)-alanyl}-*cis* ,*syn* -octahydro-1H-indole-2(S)-carboxylic acid, benzyl ester.

E. Treat the product (7.3 g) of part D with 20% HBr in glacial acetic acid (30 ml) at 0-5° and then stir at room temperature for 3 hr. Concentrate the reaction mixture in vacuo and wash the residue with ether to give the title compound, hydrobromide.

F. Treat the product (3.0 g) of part E with Bio-Rad Resin (AG 50W-X2, 100-200 mesh) in water and then add to a column of the same resin. Elute with water, then water:pyridine 96:4 and then water:pyridine: absolute ethanol 76:20:4. Concentrate the fractions (iodine positive) *in vacuo* to give the title compound.

## EXAMPLE 6

1-{N-[1(S)-Carboxy-2-[4-(6-chloro-3,4-dihydro-1,1-dioxo-7-sulfamoyl-1,2,4-benzothiadiazine-3-acetamido)-phenyl]ethyl]-(S)-alanyl}cis,syn-octahydro-1H-indole-2(S)-carboxylic acid

To the product from Example 5 (3.0 g) add 1N NaOH (20 ml) and treat as described in Example 2 to give the title compound.

## EXAMPLE 7

1-{N-[1(S)-Ethoxycarbonyl-3-[4-(6-chloro-3,4-dihydro-1,1-dioxo-7-sulfamoyl-1,2,4-benzothiadiazine-3-acetamido)phenyl]propyl]-(S)-alanyl}-cis,syn-octahydro-1H-indole-2(S)-carboxylic acid

Treat the product of Preparation 3IE as described in Example 5 to produce the title compound.

## EXAMPLE 8

1-{N-[1(S)-Carboxy-3-[4-(6-chloro-3,4-dihydro-1,1-dioxo-7-sulfamoyl-1,2,4-benzothiadiazine-3-acetamido)-phenyl]-propyl]-(S)-alanyl}cis,syn-octahydro-1H-indole-2(S)-carboxylic acid

Treat the product of Example 7 as described in Example 2 to produce the title compound.

By following the procedures described in the above preparations and examples, and by using the appropriate reagents, the following compounds may be prepared:

1-{Nα-[1(S)-ethoxycarbonyl-3-[4-(6-chloro-3,4-dihydro-1,1-dioxo-7-sulfamoyl-1,2,4-benzothiadiazine-3-acetamido)phenyl]propyl]-(S)-lysyl}-cis ,syn -octahydro-1H -indole-2(S)-carboxylic acid;

1-{N-[1(S)-methoxycarbonyl-4-[4-(6-chloro-3,4-dihydro-1,1-dioxo-7-sulfamoyl-1,2,4-benzothiadiazine-3-acetamido)phenyl]butyl]-(S)-alanyl}-cis ,syn -octahydro-1H -indole-2(S)-carboxylic acid;

2-{N-[1(S)-ethoxycarbonyl-3-[4-(6-chloro-3,4-dihydro-1,1-dioxo-7-sulfamoyl-1,2,4-benzothiadiazine-3-acetamido)phenyl]propyl]-(S)-alanyl}-1,2,3,4-tetrahydroisoquinoline-3(S)-carboxlic acid;

1-{N-[1(S)-(2-phenoxyethoxycarbonyl)-3-[4-(4-chloro-3-sulfamoylbenzamido)phenyl]propyl]-(S)-alanyl}-cis,syn-octahydro-1H-indole-2(S)-carboxylic acid;

1-{N-[1(S)-pivaloyloxymethoxycarbonyl)-3-[4-(4-chloro-3-sulfamoylbenzenesulfonamido)phenyl]propyl]-(S)-alanyl}-cis ,syn -octahydro-1H -indole-2(S)-carboxylic acid;

1-{N-[1(S)-ethoxycarbonyl-3-[4-(6-chloro-3,4-dihydro-1,1-dioxo-2-methyl-7-methylsulfamoyl-1,2,4-benzothiadiazine-3-acetamido)phenyl]propyl]-(S)-alanyl}-cis ,syn -octahydro-1H -indole-2(S)-carboxylic acid;

1-{N-[1(S)-ethoxycarbonyl-3-[4-(4-chloro-3-sulfamoyl-benzenesulfonamido)phenyl]propyl]-(S)-alanyl}-(S)-proline;

1-{N-[1(S)-carboxy-3-[4-(6-chloro-3,4-dihydro-1,1-dioxo-7-sulfamoyl-1,2,4-benzothiadiazine-3-acetamido)-phenyl]-propyl]-(S)-alanyl}-(S)-proline;

7-{N-[1(S)-carboxy-3-[4-(6-chloro-3,4-dihydro-1,1-dioxo-7-sulfamoyl-1,2,4-benzothiadiazine-3-acetamido)-phenyl]propyl]-(S)-alanyl}-1,4-dithia-7-azaspiro[4.4]nonane-8(S)-carboxylic acid;

7-{N-[1(S)-carboxy-3-[4-(4-chloro-3-sulfamoylbenzamido)phenyl]propyl]-(S)-alanyl}-1,4-dithia-7-azaspira-[4.4]-nonane-8(S)-carboxylic acid;

2-{N-[1(S)-carboxy-3-[4-(4-chloro-3-sulfamoylbenzenesulfonamido)phenyl]propyl]-(S)-alanyl}-1,2,3,4-tetrahydroisoquinoline-3(S)-carboxylic acid;

1-{N-[1(S)-ethoxycarbonyl-4-[2-(6-trifluoromethyl-3,4-dihydro-1,1-dioxo-7-sulfamoyl-1,2,4-benzothiadiazinyl-3-acetamido)phenyl]propyl]-glycyl}-cis ,syn -octahydro-1H -indole-2(S)-carboxylic acid;

1-{N-[1(S)-ethoxycarbonyl-2-[4-(6-chloro-3,4-dihydro-2-benzyl-1,1-dioxo-7-sulfamoyl-1,2,4-benzothiadiazine-3-acetamido)phenylthio]ethyl]-(S)-alanyl}-cis ,syn -octahydro-1H -indole-2(S)-carboxylic acid;

1-{N-[1(S)-ethoxycarbonyl-2-[4-(6-chloro-3,4-dihydro-1,1-dioxo-7-sulfamoyl-1,2,4-benzothiadiazine-2-benzyl-3-acetamido)phenyl]methoxyethyl]-(S)-alanyl}-cis ,syn -octahydro-1H -indole-2(S)-carboxylic acid;

1-{N-[1(S)-ethoxycarbonyl-3-[4-(6-chloro-3,4-dihydro-1,1-dioxo-7-sulfamoyl-1,2,4-benzothiadiazin-3-yl)-methylthiophenyl]propyl]-(S)-alanyl}-cis ,syn -octahydro-1H -indole-2(S)-carboxylic acid;

1-[N-[1(S)-carboxy-3-[4-(4-chloro-3-sulfamoyl benzamido)phenyl]propyl]-(S)-alanyl]-cis,syn-octahydroindole-2-(S)-carboxylic acid; $[\alpha]_D^{26} = -1.5^\circ$ MeOH

1-[N-[1(S)-carboxy-3-[4-(4-chloro-3-N-methyl-sulfamoyl benzamido)phenyl]propyl]-(S)-alanyl]-cis,syn-octahydroindole-2(S)-carboxylic acid; $[\alpha]_D^{26} = -1.2^\circ$ MeOH

The compounds of this invention are useful in view of their pharmacological properties. In particular, they possess activity as antihypertensive agents, as evidenced by their ability to reduce blood pressure in

mammals in which the blood pressure has become abnormally elevated.

Since these compounds are believed to act as angiotensin converting enzyme inhibitors, it is also contemplated that they may be used in treating other cardiovascular disorders, for example congestive heart failure, and glaucoma in the same manner as other ACE inhibitors such as captopril and enalapril may be used.

The compounds of this invention can be combined with pharmaceutical carriers and administered in a variety of well-known pharmaceutical forms suitable for oral transdermal or parenteral administration to provide compositions useful in the treatment of cardiovascular disorders and particularly mammalian hypertension.

The effective daily antihypertensive dose (ED50) of the compounds of this invention will typically be in the range of about 0.1 to about 25 mg/kg, of mammalian weight, administered in single or divided doses. The exact dose to be administered is determined by the attending clinician and is dependent upon where the particular compound lies within the above quoted range, as well as upon the age, weight and condition of the individual.

Generally, in treating humans having hypertension, the compounds of this invention may be administered to patients in need of such treatment in a dosage range of about 5 to about 500 mg per patient generally given several times a day, thus giving a total daily dose of from about 5 to about 2000 mg per day.

The antihypertensive compositions containing the compounds of this invention will preferably contain from about 5 to about 250 mg of the active compound per dosage unit.

The compositions of the present invention are most preferably administered orally. Typical formulations for oral administration are those such as tablets, capsules, syrups, elixirs or suspensions. Typical injectable formulations include solutions and suspensions. Also contemplated are mechanical delivery systems, e.g. transdermal dosage forms.

The typical acceptable pharmaceutical carriers for use in the formulations described above are exemplified by: sugars such as lactose, sucrose, mannitol and sorbitol; starches such as corn starch, tapioca starch and potato starch; cellulose and derivatives such as sodium carboxymethyl cellulose, ethyl cellulose and methyl cellulose; calcium phosphates such as dicalcium phosphate and tricalcium phosphate; sodium sulfate; calcium sulfate; polyvinylpyrrolidone, polyvinyl alcohol; stearic acid; alkaline earth metal stearates such as magnesium stearate and calcium stearate, stearic acid, vegetable oils such as peanut oil, cottonseed oil, sesame oil, olive oil and corn oil; non-ionic, cationic and anionic sufactants; ethylene gylcol polymers; betacyclodextrin; fatty alcohols and hydrolyzed cereal solids; as well as other non-toxic compatible fillers, binders, disintegrants, buffers, preservatives, anti-oxidants, lubricants, flavoring agents, and the like commonly used in pharmaceutical formulations.

The active compounds of the invention (ACE inhibitors) are administered in the form of ophthalmic pharmaceutical compositions adapted for topical administration to the eye; such as solutions, suspensions, ointments and solid inserts. Formulations of these compounds may contain from 0.00001 to 1% and especially 0.001 to 1% of medicament. Other concentrations may be employed provided the dose is effective in lowering intraocular pressure. As a unit dosage from, between 0.01μg to 1.0 mg., and especially 1.0 to 100 μg of the active compound is applied to the human eye, generally on a daily basis. Individual dosage requirements are variable, however, and must be administered on the basis of the severity of the disease and the response of the patient.

To prepare suitable dosage forms, the active compounds may be conveniently admixed with a non-toxic pharmaceutically acceptable carrier suitable for topical ophthalmolgic administration. Typical of such pharmaceutically acceptable carriers are, for example, water, mixtures of water and watermiscible solvents such as lower alkanols or vegetable oils, petroleum based jelly, and including also from 0.5 to 5% by weight of hyroxyethyl cellulose, ethyl oleate, carboxymethyl cellulose, polyvinylpyrrolidone, and other water soluble ophthalmologically acceptable non-toxic polymers, for example, cellulose derivatives such as methyl cellulose, alkali metal carboxymethyl cellulose, hydroxyethyl cellulose, hydroxypropyl cellulose, hydroxypropylmethyl cellulose; acrylates such as polyacrylic acids salts, ethylacrylates; polyacrylamides; natural products such as gelatin, alginates, pectins, tragacanth, karaya, chondrus, agar, acacia; the starch derivatives such as starch acetate, hydroxyethyl starch ethers, hydroxypropyl starch, as well as other synthetic derivatives such as polyvinyl alcohol, polyvinyl pyrrolidone-polyvinyl methyl ether, polyethylene oxide, neutralized carbopol and xanthan gum and mixtures of these polymers. The pharmaceutical preparation may also contain non-toxic auxiliary substances such as emulsifying, preserving, wetting, bodying agents and the like, as for example, polyethylene glycols 200, 300, 400 and 600, carbowaxes 1,000, 1,500, 4,000, 6,000 and 10,000, antibacterial components such as quaternary ammonium compounds; phenylmercuric salts known to have cold sterilizing properties and which are non-injurious in use; thimerosal, methyl and

propyl paraben, benzyl alcohol, phenyl ethanol; buffering ingredients such as alkali metal chloride, borate, acetate, gluconate buffers; antioxidants such as sodium metabisulfite, butylated hydroxyanisole (BHA), butylated hydroxytoluene (BHT) and the like; and other conventional ingredients such as sorbitan monolaurate, triethanolamine, oleate, polyoxyethylene sorbitan monopalmitylate, dioctyl alkali metal sulfosuccinate, monothioglycerol, ethylenediamine tetracetic acid and the like.

Additionally, suitable ophthalmic vehicles can be used as carrier media for the present purpose including conventional phosphate buffer vehicle systems, isotonic boric acid vehicles, isotonic alkali chloride vehicles, tris and the like.

The pharmaceutical preparation may also be in the form of a solid insert. For example, one may use a solid water soluble polymer as the carrier for the medicament. Inserts that are known in the art that are suitable for this use include those described in British Patent 15611, and in United States Patents 3,993,071; 3,986,510; 3,868,445; and 3,867,510. Solid water insoluble inserts, such as those prepared from ethylene vinyl acetate copolymer, may also be utilized.

The compositions of the invention may include additional therapeutic agents in addition to the ACE inhibitor. For example antibiotics, anesthetics as well as other IOP lowering agents may be present.

In the following examples, the "active ingredient" is 1-{N-[1(S)-ethoxycarbonyl-3-[4-(6-chloro-3,4-dihydro-1,1-dioxo-7-sulfamoyl-2H -1,2,4-benzothiadiazine)acetamido]phenyl]propyl]-(S)-alanyl}cis ,syn -octahydro-1H-indole-2(S)-carboxylic acid. It is contemplated, however, that this compound may be replaced by equally effective quantities of other compounds within the scope of formula I.

## EXAMPLE 10

| Capsule | Amount (mg) | |
|---|---|---|
| Active ingredient | 250.0 | 125.0 |
| Lactose | 173.0 | 86.5 |
| Corn Starch | 75.0 | 37.5 |
| Magnesium stearate | 2.0 | 1.0 |
| | 500.0 | 250.0 |

Blend the active ingredient, lactose, and corn starch until uniform; then blend the magnesium stearate into the resulting powder. Encapsulate the mixture into suitably sized two-piece hard gelatin capsules.

## EXAMPLE 11

| Tablet | Amount (mg) | |
|---|---|---|
| Active Ingredient | 250.0 | 125.0 |
| Lactose | 161.0 | 80.5 |
| Corn Starch | 12.0 | 6.0 |
| Water (per thousand tablets) | 120 ml (evaporates) | 60 ml (evaporates) |
| Corn Starch | 75.0 | 37.5 |
| Magnesium Stearate | 2.0 | 1.0 |
| | 500.0 | 250.0 |

Blend the active ingredient with the lactose until uniform. Blend the smaller quantity of corn starch with the water and add the resulting corn starch paste, then mix until a uniform wet mass is formed. Add the remaining corn starch to the remaining wet mass and mix until uniform granules are obtained. Screen the granules through a suitable milling machine, using a 3/4 inch stainless steel screen. Dry the milled granules in a suitable drying oven until the desired moisture content is obtained. Mill the dried granules through a suitable milling machine using a 16 mesh stainless steel screen. Blend in the magnesium stearate and compress the resulting mixture into tablets of desired shape, thickness, hardness and disintegration.

## EXAMPLE 12

| Injectable Solution | mg/ml |
|---|---|
| Active ingredient | 5.00 |
| Methyl *p*-hydroxybenzoate | 0.80 |
| Propyl *p*-hydroxybenzoate | 0.10 |
| Disodium Edetate | 0.10 |
| Citric Acid Monohydrate | 0.08 |
| Dextrose | 40.0 |
| Water for injection qs. ad. | 1.0 ml |

Dissolve the p -hydroxybenzoates in a portion of water for injection at 60-70 °C and cool the solution to 25-25 °C. Charge and dissolve all other excipients and the active ingredient. Bring the solution to final volume, filter it through a sterilizing membrane and fill into sterile containers.

Example 13:

Ophthalmic Compositions:

a) Ophthalmic Solution

| | mg/ml |
|---|---|
| active ingredient | * |
| Polyvinyl Alcohol | 20.0 |
| Sodium phosphate Dibasic | 1.2 |
| Sodium phosphate Monobasic | 0.64 |
| Edetate Disodium | 0.1 |
| Sodium Chloride | 6.0 |
| Benzalkonium Chloride | 0.1 |
| Purified Distilled Water QS. A.D. | 1.0ml |

b) Ophthalmic Solution

| | mg/ml |
|---|---|
| active ingredient | * |
| Hydroxypropyl Methylcellulose | 5.0 |
| Boric acid | 10.0 |
| Benzalkonium Chloride | 0.1 |
| Sodium Borate | 0.7 |
| Edetate Disodium | 0.1 |
| Sodium Chloride | 3.0 |
| Purified Distilled Water QS.A.D. | 1.0ml |

c) Ophthalmic Ointment:

| | µg/g. |
|---|---|
| active ingredient | * * |
| Purified Distilled Water | 0.1ml |
| Methyl Paraben | 0.8 |
| Propyl Paraben | 0.1 |
| Hydrophilic Petrolatum QS. A.D. | 1.0g |

d) Ophthalmic Ointment

| | µg/g. |
|---|---|
| active ingredient | * * |
| Chlorobutanol | 5 |
| Anhydous lanolin | 10 |
| Mineral Oil | 10 |
| White Petrolatum QS. A.D. | 1.0g |

e) Opthalmic jel:

| | µg/ml |
|---|---|
| active ingredient | * * |
| Hydroxypropyl Methylcellulose | 40.0 |
| Boric Acid | 10.0 |
| Benzalkonium Chloride | 0.1 |
| Sodium Borate | 0.7 |
| Edetate Disodium | 0.1 |
| Purified Distilled Water QS. A.D. | 1.0ml |

* stands for the concentration of the active ingredient which is 0.0001-10.0 mg/ml,

** stands for the concentration of the active ingredient which is 0.0001 - 10.0 mg/g.

Similarly, substitute other compounds of the present invention to prepare other compositions of the present invention.

As mentioned before the compounds of this invention show valuable antihypertensive activity. It can be concluded from animal tests that these compounds have high angiotensin converting enzyme inhibitory activity (ACE inhibitory activity).

The in-vivo ACE inhibitory activity of

Compound A:

1- [N-[1(S)-carboxy-3- [4-(4-chloro-3-sulfamoyl benzamido) phenyl] propyl](S)-alanyl]cis,syn-octahydroindole-2-(S)-carboxylic acid

Compound B:

1-[N-[1(S)-carboxy-3-[4-(4-chloro-3-N-methyl-sulfamoyl benzamido)phenyl]propyl]-(S)-alanyl]cis, syn-octahydroindole-2(S)-carboxylic acid is as follows:

| | $ID_{50}$ (i.v.) | $ID_{50}$ (oral) |
|---|---|---|
| Compound A | 36 µg/kg | 3 mg/kg |
| Compound B | 33 µg/kg | 3 mg/kg |

$ID_{50}$: Dose (intravenous or oral respectively) required to inhibit the pressor response to i.v. angiotensin I by 50% in anesthethised rats.

## Claims

**1.** A compound represented by the formula:

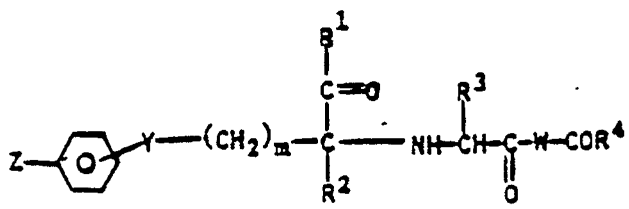 I

wherein W is

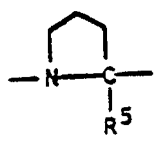 , 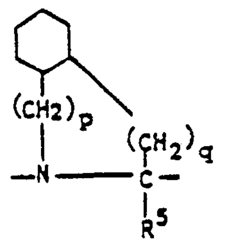 , 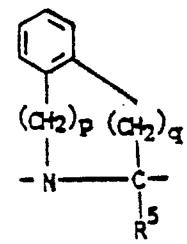 , 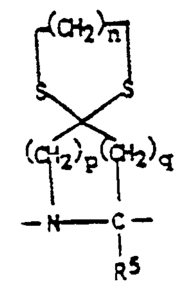 ;

II III IV V

n is 0 or 1; m is 0 to 2;
p and q are each 0, 1 or 2, provided that the sum of p and q is 1 or 2, and that in formula V, p is not 0;
Y is $-CH_2-$, $-CH_2O-$, or $-CH_2S-$, attached at the 2 or 4 position of phenyl group;
Z is

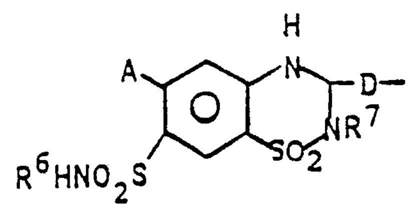 , 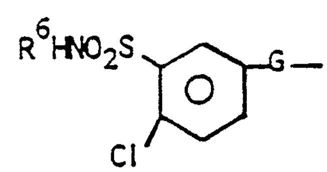

VI VII

wherein
A is Cl or $CF_3$;
D is $-(CH_2)_u-$, $-CH_2O-$, $-CH_2S-$;

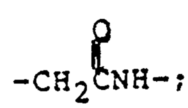

G is $-CONR^7(CH_2)_t-$, or $-SO_2NR^7(CH_2)_t-$; t is 0 or 1;
$R^1$ and $R^4$ are independently hydroxy, alkoxy having from 1 to 8 carbon atoms, $K-X_r-(CH_2)_s-O-$, wherein K is phenyl, substituted phenyl, 1- or 2-naphthyl, X is oxygen or sulfur, r is 0 or 1 and s is 0 to 4, and wherein the substitutents on the phenyl are chosen from group M, wherein M is halogen, hydroxy, trifluoromethyl, alkoxy having 1 to 6 carbon atoms, 2- and 3-furanyl, 2-and 3-thienyl and phenyl (which phenyl group may be substituted with halogen, hydroxy, trifluoromethyl, alkoxy having from 1 to 6 carbon atoms or alkyl having from 1 to 6 carbon atoms), provided that when s is zero, r is zero, $-OCH_2OCO-$alkyl wherein the alkyl has from 3 to 8 carbon atoms, $-OCH_2CO-$phenyl, wherein the phenyl may be substituted with group M, 1-glyceryl,

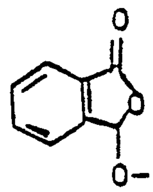
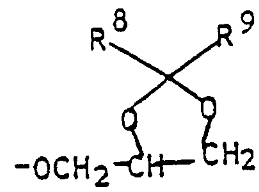

$R^2$, $R^5$, $R^6$ and $R^9$ are hydrogen or $C_1$-$C_6$ alkyl;
$R^3$ is hydrogen, $C_1$-$C_6$ alkyl or amino $C_1$-$C_6$ alkyl;
$R^7$ is hydrogen, $C_1$-$C_6$ alkyl or phenyl($C_1$-$C_6$)alkyl;
$R^8$ is hydrogen, $C_1$-$C_6$ alkyl, phenyl, or phenyl substituted by group M;
u is 1 or 2;
and the pharmaceutically acceptable salts thereof.

2. A compound of claim 1 wherein W is represented by formula III, IV or V.

3. A compound of claim 1 or claim 2 wherein W is represented by formula III or IV, wherein p is 0 and q is 1.

4. A compound of any of claims 1-3 above wherein $R^2$ and $R^5$ are hydrogen.

5. A compound of any of claims 1-4 above wherein $R^4$ is hydroxy.

6. A compound of any of claims 1-5 above wherein $R^3$ is methyl or aminobutyl.

7. A compound of any of claims 1-6 above wherein A is chlorine, and $R^6$ and $R^7$ are hydrogen or methyl.

8. A compound of any of claims 1-7 wherein $R^1$ is hydroxy, ethoxy, methoxy, phenoxyethoxy, or pivaloyloxymethoxy.

9. A compound as defined in claim 1 which is:
(1) 1-{N-[1-(S)-ethoxycarbonyl-2-[4-(3-sulfamoyl-4-chlorobenzamido)-phenyl]ethyl]-(S)alanyl}-cis ,syn -octahydro-1H-indole-2(S)-carboxylic acid;
(2) 1-{N-[1-(S)-ethoxycarbonyl-2-[4-(4-chloro-3-N-methylsulfamoyl-benzamido)phenyl]ethyl]-(S)-alanyl}-cis ,syn -octahydro-1H -indole-2(S)-carboxylic acid;
(3) 1-[N-[1(S)-carboxy-2-[4-(4-chloro-3-sulfamoylbenzamido)phenyl]-ethyl]-(S)-alanyl}-cis ,syn -octahydro-1H -indole-2(S)-carboxylic acid;
(4) 1-{N-[1-(S)-carboxy-2-[4-(4-chloro-3-N-methylsulfamoylbenzamido)-phenyl]ethyl]-(S)-alanyl}-cis ,syn -octahydro-1H -indole-2(S)-carboxylic acid;
(5) 1-{N-[1-(S)-ethoxycarbonyl-3-[4-(4-chloro-3-sulfamoylbenzamido)-phenyl]propyl]-(S)-alanyl}-cis ,syn -octahydro-1H -indole-2(S)-carboxylic acid;
(6) 1-{N-[1-(S)-ethoxycarbonyl-3-[4-(4-chloro-3-N-methylsulfamoyl-benzamido)phenyl]propyl]-(S)-alanyl}-cis ,syn -octahydro-1H -indole-2(S)-carboxylic acid; or
(7) 1-{N-[1-(S)-ethoxycarbonyl-3-[4-(6-chloro-3,4-dihydro-1,1-dioxo-7-sulfamoyl-2H-1,2,4-benzothiadiazine-3-acetamido)-phenyl]propyl]-(S)-alanyl}-cis ,syn -octahydro-1H -indole-2(S)-carboxylic acid.

10. Process for the preparation of a compound of formula I characterized in that
(a) an amino acid of formula VIII is condensed with a ketocompound of formula IX in the presence of a reducing agent in a solvent

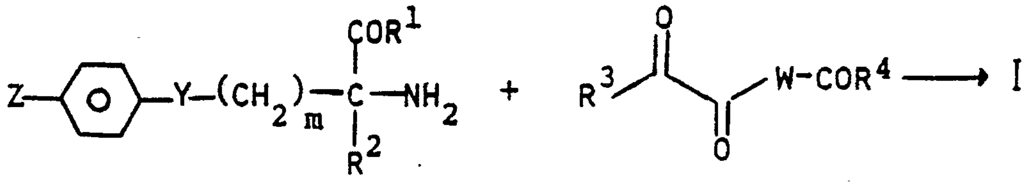

VIII IX

wherein Z, Y, $R^1$, $R^2$, $R^3$, $R^4$, m and W are as defined above;
(b) for the preparation of compounds of formula I wherein Y is $-CH_2-$, and Z is a group of formula VII, an acid of formula XIX is reacted with an amine of formula XX:

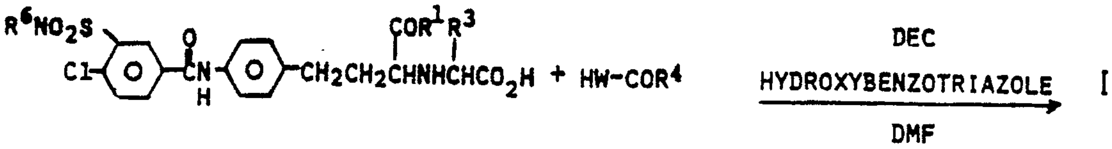


XIX XX

wherein $R^1$, $R^3$, $R^4$, $R^6$ and W are as defined above, and "DEC" refers to 1-(3-dimethylaminopropyl)-3-ethyl carbodiimide HCl;
(c) for the preparation of compounds of formula I wherein Y is $-CH_2-$, and Z is a group of formula VII, an acid chloride of formula XXVII is reacted with a dipeptide of formula XXVIII:

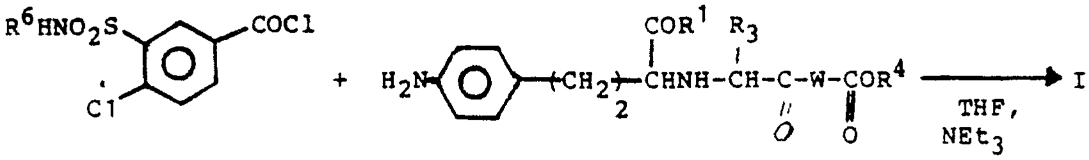


XXVII XXVIII

wherein $R^1$, $R^3$, $R^4$, $R^6$ and W are as defined above;
followed, if desired by (i) salt formation and/or (ii) esterification or transesterification and/or de-esterification and/or (iii) isolation of isomers.

**11.** A pharmaceutical composition comprising as active ingredient at least one compound as claimed in any one of claims 1-10 together with a pharmaceutical carrier and/or excipient.

**12.** A method for making a pharmaceutical composition comprising mixing a compound of formula I with a pharmaceutically acceptable carrier.

**13.** The use of a compound of formula I for the preparation of a medicament for the treatment of hypertension.

**14.** The use of a compound of formula I for the preparation of a medicament for the treatment of glaucoma.

Claim for the following Contracting State: AT

**1.** A method of preparing a compound having the formula:

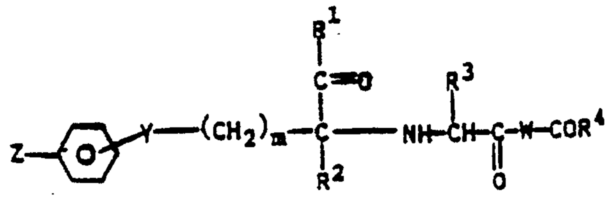

I

wherein W is

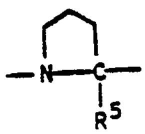
,
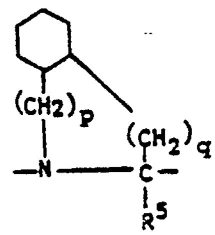
,
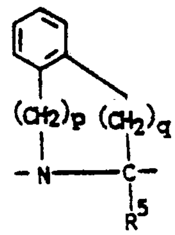
,
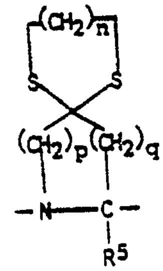
;

II III IV V

n is 0 or 1; m is 0 to 2;
p and q are each 0, 1 or 2, provided that the sum of p and q is 1 or 2, and that in formula V, p is not 0;
Y is $-CH_2-$, $-CH_2O-$, or $-CH_2S-$, attached at the 2 or 4 position of phenyl group;
Z is

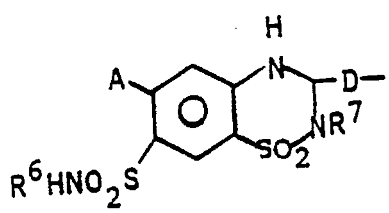
,
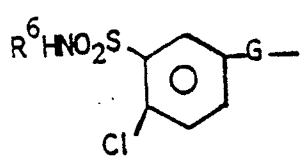

VI VII

wherein
A is Cl or $CF_3$;
D is $-(CH_2)_u-$, $-CH_2O-$, $-CH_2S-$;

$$-CH_2\overset{\overset{O}{\|}}{C}NH-;$$

G is $-CONR^7(CH_2)_t-$, or $-SO_2NR^7(CH_2)_t-$; t is 0 or 1;
$R^1$ and $R^4$ are independently hydroxy, alkoxy having from 1 to 8 carbon atoms, $K-X_r-(CH_2)_s-O-$, wherein K is phenyl, substituted phenyl, 1- or 2-naphthyl, X is oxygen or sulfur, r is 0 or 1 and s is 0 to 4, and wherein the substituents on the phenyl are chosen from group M, wherein M is halogen, hydroxy, trifluoromethyl, alkoxy having 1 to 6 carbon atoms, 2- and 3-furanyl, 2-and 3-thienyl and phenyl (which phenyl group may be substituted with halogen, hydroxy, trifluoromethyl, alkoxy having from 1 to 6 carbon atoms or alkyl having from 1 to 6 carbon atoms), provided that when s is zero, r is zero, $-OCH_2OCO$-alkyl wherein the alkyl has from 3 to 8 carbon atoms, $-OCH_2CO$-phenyl, wherein the phenyl may be substituted with group M, 1-glyceryl,

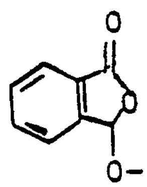

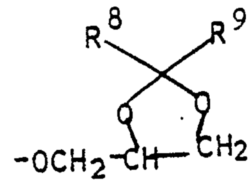

$R^2$, $R^5$, $R^6$ and $R^9$ are hydrogen or $C_1$-$C_6$ alkyl;
$R^3$ is hydrogen, $C_1$-$C_6$ alkyl or amino $C_1$-$C_6$ alkyl;
$R^7$ is hydrogen, $C_1$-$C_6$ alkyl or phenyl($C_1$-$C_6$) alkyl;
$R^8$ is hydrogen, $C_1$-$C_6$ alkyl, phenyl, or phenyl substituted by group M;
u is 1 or 2;
and the pharmaceutically acceptable salts thereof characterized in that

(a) an amino acid of formula VIII is condensed with a ketocompound of formula IX in the presence of a reducing agent in a solvent

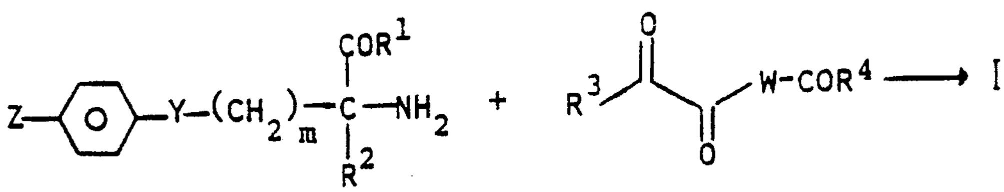

VIII IX

wherein Z, Y, $R^1$, $R^2$, $R^3$, $R^4$, m and W are as defined above;

(b) for the preparation of compounds of formula I wherein Y is $-CH_2-$, and Z is a group of formula VII, an acid of formula XIX is reacted with an amine of formula XX:

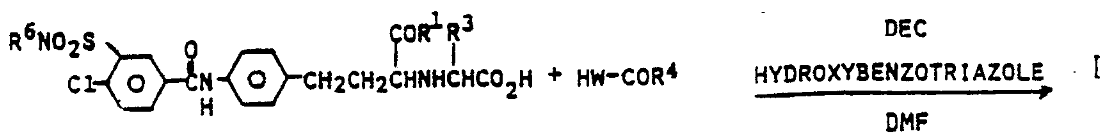


XIX XX

wherein $R^1$, $R^3$, $R^4$, $R^6$ and W are as defined above, and "DEC" refers to 1-(3-dimethylaminopropyl)-3-ethyl carbodiimide HCl;

(c) for the preparation of compounds of formula I wherein Y is $-CH_2-$, and z is a group of formula VII, an acid chloride of formula XXVII is reacted with a dipeptide of formula XXVIII:

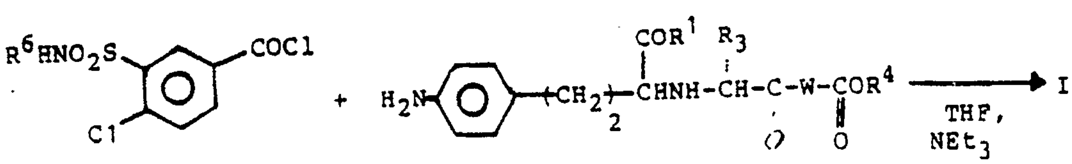


XXVII XXVIII

wherein $R^1$, $R^3$, $R^4$, $R^6$ and W are as defined above;
followed, if desired by (i) salt formation and/or (ii) esterification or transesterification and/or de-esterification and/or (iii) isolation of isomers.

2. A method according to claim 1 in which a compound is produced wherein W is represented by formula III, IV or V.

3. A method according to claims 1 or 2 above in which a compound is produced wherein W is represented by formula III or IV, wherein p is 0 and q is 1.

4. A method according to claims 1 or 2 above in which a compound is produced wherein W is represented by formula V, wherein p is 1, q is 1, and n is 0.

5. A method according to any of claims 1-4 above in which a compound is produced wherein $R^2$ and $R^5$ are hydrogen.

6. A method according to any of claims 1-5 above in which a compound is produced wherein $R^4$ is hydroxy.

7. A method according to any of claims 1-6 above in which a compound is produced wherein $R^3$ is methyl or aminobutyl.

8. A method according to any of claims 1-7 above in which a compound is produced wherein A is chlorine, and $R^6$ and $R^7$ are hydrogen or methyl.

9. A method according to any of claims 1-8 above in which a compound is produced wherein $R^1$ is hydroxy, ethoxy, methoxy, phenoxyethoxy, or pivaloyloxymethoxy.

10. A method according to any of claims 1-9 above in which the compound is:
1-{N-[1-(S)-ethoxycarbonyl-2-[4-(3-sulfamoyl-4-chlorobenzamido)-phenyl]ethyl]-(S)alanyl}-cis ,syn -octahydro-1H -indole-2(S)-carboxylic acid;
1-{N-[1-(S)-ethoxycarbonyl-2-[4-(4-chloro-3-N-methylsulfamoyl-benzamido)phenyl]ethyl]-(S)alanyl}-cis ,syn -octahydro-1H -indole-2(S)-carboxylic acid;
1-{N-[1(S)-carboxy-2[4-(4-chloro-3-sulfamoylbenzamido)phenyl]-ethyl]-(S)-alanyl}-cis ,syn -octahydro-1H -indole-2(S)-carboxylic acid;
1-{N-[1-(S)-carboxy-2-[4-(4-chloro-3-N-methylsulfamoylbenzamido)-phenyl]ethyl]-(S)-alanyl}-cis ,syn -octahydro-1H -indole-2(S)-carboxlic acid;
1-{N-[1-(S)-ethoxycarbonyl-3-[4-(4-chloro-3-sulfamoylbenzamido)-phenyl]propyl]-(S)-alanyl}-cis ,syn -octahydro-1H -indole-2(S)-carboxylic acid;
1-{N-[1-(S)-ethoxycarbonyl-3-[4-(4-chloro-3-N-methylsulfamoyl-benzamido)phenyl]propyl]-(S)-alanyl}-cis ,syn -octahydro-1H-indole-2(S)-carboxylic acid;
1-{N-[1-(S)-ethoxycarbonyl-3-[4-(6-chloro-3,4-dihydro-1,1-dioxo-7-sulfamoyl-2H -1,2,4-benzothiadiazine-3-acetamido)-phenyl]propyl]-(S)-alanyl}-cis ,syn -octahydro-1H -indole-2(S)-carboxylic acid; or a pharmaceutically acceptable salt.

11. A method of producing a pharmaceutical composition which comprises mixing a compound of formula I as defined in claim 1, or salt thereof with a pharmaceutically acceptable carrier or excipient.

12. A method of producing a pharmaceutical composition which comprises mixing a compound of formula I or a salt thereof, prepared by a process of any one of Claims 1 to 10, with a pharmaceutically acceptable carrier or excipient.

13. A method for producing a pharmaceutical composition useful in the treatment of glaucoma and/or hypertension characterized by admixing a compound of formula I as defined in claim 1 or a salt thereof with a pharmaceutically acceptable carrier or excipient.

## Revendications

1. Composé représenté par la formule :

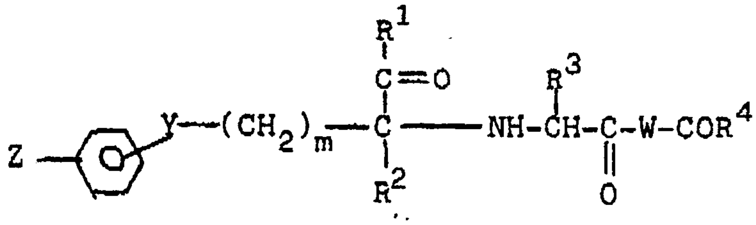

I

où W est

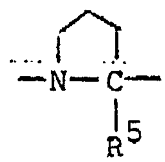 , 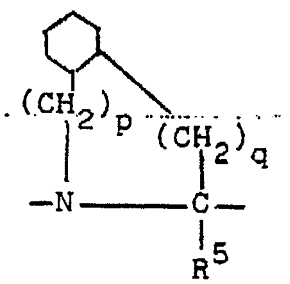 , 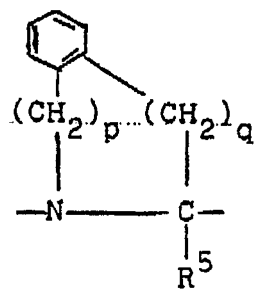 , 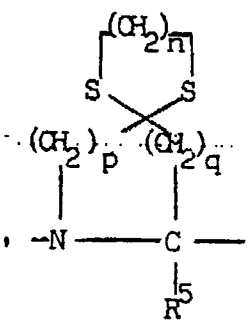 ;

II III IV V

n est 0 ou 1 ; m est 0 à 2 ;
chacun de p et q est 0, 1 ou 2, à condition que la somme de p et q soit 1 ou 2 et que dans la formule V, p ne soit pas 0 ;
Y est $-CH_2-$, $-CH_2O-$, ou $-CH_2S-$, attaché à la position 2 ou 4 du groupe phényle ;
Z est

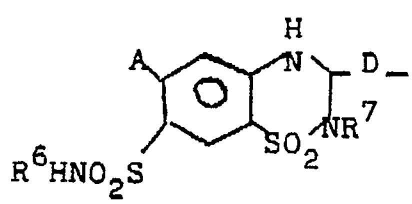 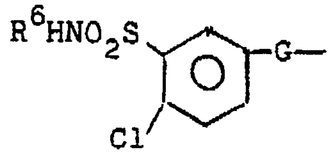

VI VII

où A est Cl ou $CF_3$ ;
D est $-(CH_2)_u-$, $-CH_2O-$, $-CH_2S-$ ;

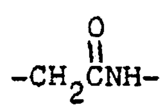 ;

G est $-CONR^7(CH_2)_t-$, ou $SO_2NR^7(CH_2)_t-$ ; t est 0 ou 1 ;
$R^1$ et $R^4$ sont indépendamment hydroxy, alcoxy ayant de 1 à 8 atomes de carbone, $K-X_r-(CH_2)_s-O-$, où K est phényle, phényle substitué, 1- ou 2-naphtyle, X est oxygène ou soufre, r est 0 ou 1 et s est 0 à 4, et où les substituants sur le phényle sont choisis dans le groupe M, où M est halogène, hydroxy, trifluorométhyle, alcoxy ayant 1 à 6 atomes de carbone, 2- et 3-furanyle, 2- et 3-thiényle et phényle (lequel groupe phényle peut être substitué par halogène, hydroxy, trifluorométhyle, alcoxy ayant 1 à 6 atomes de carbone ou alkyle ayant 1 à 6 atomes de carbone) à condition que quand s est zéro, r est zéro, $-OCH_2OCO$-alkyle où l'alkyle a 3 à 8 atomes de carbone, $-OCH_2CO$-phényle, où le phényle peut être substitué par le groupe M, 1-glycéryle,

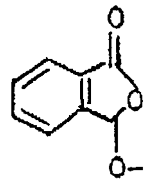 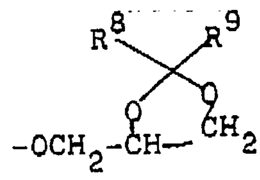

$R^2$, $R^5$, $R^6$ et $R^9$ sont hydrogène ou alkyle $C_1$-$C_6$ ;
$R^3$ est hydrogène, alkyle $C_1$-$C_6$ ou amino alkyle $C_1$-$C_6$ ;
$R^7$ est hydrogène, alkyle, $C_1$-$C_6$ où phényl-alkyle $C_1$-$C_6$ ;
$R^8$ est hydrogène, alkyle $C_1$-$C_6$, phényle, ou phényle substitué par le groupe M ;
u est 1 ou 2 ;
et ses sels acceptables en pharmacie.

2. Composé de la revendication 1, où W est représenté par la formule III, IV ou V.

3. Composé de la revendication 1 ou de la revendication 2, où W est représenté par la formule III ou IV, où p est 0 et q est 1.

4. Composé selon l'une quelconque des revendications 1-3 ci-dessus, où $R^2$ et $R^5$ sont hydrogène.

5. Composé selon l'une quelconque des revendications 1-4 ci-dessus, où $R^4$ est hydroxy.

6. Composé selon l'une quelconque des revendications 1-5 ci-dessus, où $R^3$ est méthyle ou aminobutyle.

7. Composé selon l'une quelconque des revendications 1-6 ci-dessus, où A est chlore, et $R^6$ et $R^7$ sont hydrogène ou méthyle.

8. Composé selon l'une quelconque des revendications 1-7 où $R^1$ est hydroxy, éthoxy, méthoxy, phénoxyéthoxy, ou pivaloyloxyméthoxy.

9. Composé tel que défini à la revendication 1, qui est:
(1) acide 1-{N-[1-(S)-éthoxycarbonyl-2-[4-(3-sulfamoyl-4-chlorobenzamido)-phényl]éthyl]-(S)-alanyl}-cis ,syn -octahydro-1H-indole-2(S)-carboxylique ;
(2) acide 1-{N-[1-(S)-éthoxycarbonyl-2-[4-(4-chloro-3-N-méthylsulfamoyl-benzamido)phényl]éthyl]-(S)alanyl}-cis ,syn --octahydro-1H -indole-2(S)-carboxylique ;
(3) acide 1-{N-[1(S)-carboxy-2[4-(4-chloro-3-sulfamoylbenzamido)phényl]-éthyl]-(S)-alanyl}-cis ,syn -octahydro-1H -indole-2(S)-carboxylique ;
(4) acide 1-{N-[1-(S)-carboxy-2-[4-(4-chloro-3-N-méthylsulfamoylbenzamido)-phényl]éthyl]-(S)-alanyl}-cis ,syn -octahydro-1H -indole-2(S)-carboxylique ;
(5) acide 1-{N-[1-(S)-éthoxycarbonyl-3-[4-(4-chloro-3-sulfamoylbenzamido)-phényl]propyl]-(S)-alanyl}-cis ,syn , octahydro-1H -indole-2(S)-carboxylique ;
(6) acide 1-{N-[1-(S)-éthoxycarbonyl-3-[4-(4-chloro-3-N-méthylsulfamoyl-benzamido)phényl]propyl]-(S)-alanyl}-cis ,syn -octahydro-1H -indole-2(S)-carboxylique ; ou
(7) acide 1-{N-[1-(S)-éthoxycarbonyl-3-[4-(6-chloro-3,4-dihydro-1,1-dioxo-7-sulfamoyl-2H -1,2,4-benzothiadiazine-3-acétamido)-phényl]propyl]-(S)-alanyl}-cis ,syn -octahydro-1H -indole-2(S)-carboxylique.

10. Procédé pour la préparation d'un composé de formule I, caractérisé en ce que
(a) un acide aminé de formule VIII est condensé avec un composé céto de formule IX en présence d'un agent réducteur dans un solvant

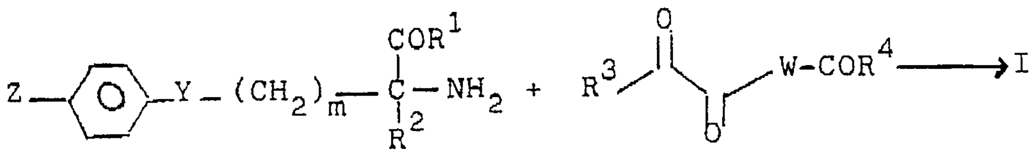

VIII IX

où Z, Y, $R^1$, $R^2$, $R^3$, $R^4$, m et W sont tels que définis ci-dessus ;

(b) pour la préparation des composés de formule I où Y est $-CH_2-$, et Z est un groupe de formule VII, on fait, réagir un acide de formule XIX avec une amine de formule XX :

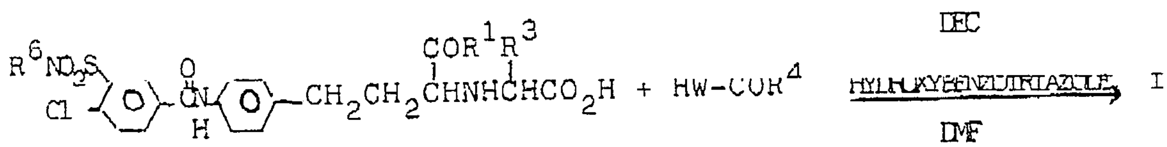


XIX XX

où $R^1$, $R^3$, $R^4$, $R^6$ et W sont tels que définis ci-dessus, et "DEC" indique 1-(3-diméthylaminopropyl)-3-éthyl carbodiimide HCl ;

(c) pour la préparation des composés de formule I où Y est $-CH_2-$, et Z est un groupe de formule VII on fait réagir un chlorure d'acide de la formule XXVII avec un dipeptide de la formule XXVIII :

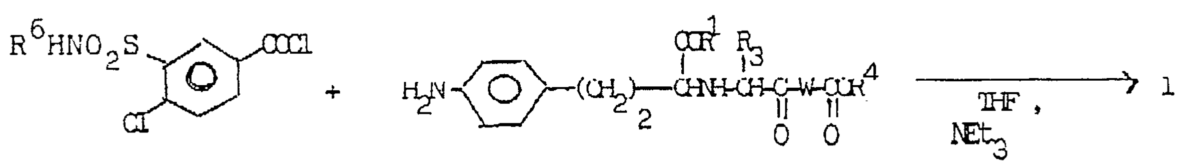


XXVII XXVIII

où $R^1$, $R^3$, $R^4$, $R^6$ et W sont tels que définis ci-dessus avec ensuite, si on le souhaite, (1) la formation d'un sel et/ou (II) l'estérification ou la transestérification et/ou la desestérification et/ou (iii) l'isolement des isomères.

**11.** Composition pharmaceutique comprenant, comme ingrédient actif au moins un composé selon l'une quelconque des revendications 1-10 avec un véhicule et/ou excipient pharmaceutique.

**12.** Méthode de fabrication d'une composition pharmaceutique comprenant le mélange d'un composé de formule I avec un véhicule acceptable en pharmacie.

**13.** Utilisation d'un composé de formule I pour la préparation d'un médicament pour le traitement de l'hypertension.

**14.** Utilisation d'un composé de formule I pour la préparation d'un médicament pour le traitement du glaucome.

Revendications pour l'Etat contractant suivant: AT

**1.** Méthode de préparation d'un composé ayant pour formule :

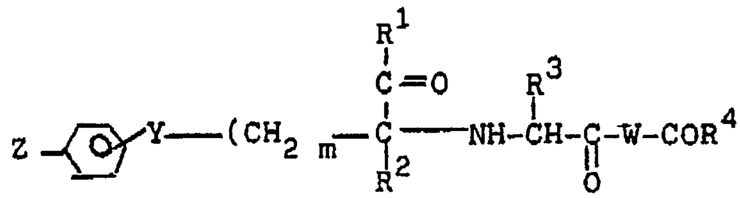

I

où W est

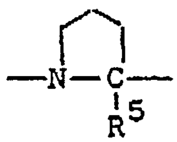

,

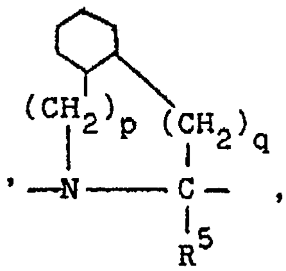

,

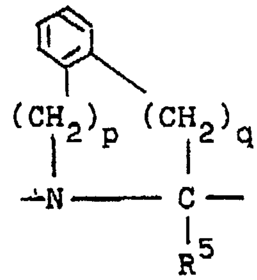

,

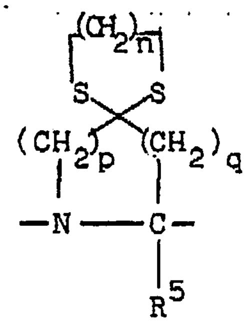

;

II III IV V

n est 0 ou 1 ; m est 0 à 2 ;

chacun de p et q est 0, 1 ou 2, à condition que la somme de p et q soit 1 ou 2 et que dans la formule V, p ne soit pas 0 ;

Y est $-CH_2-$, $-CH_2O-$, ou $-CH_2S-$, attaché à la position 2 ou 4 du groupe phényle ;

Z est

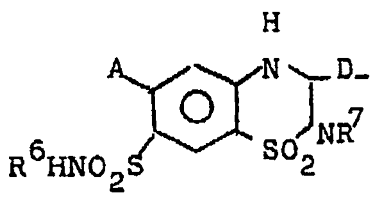

,

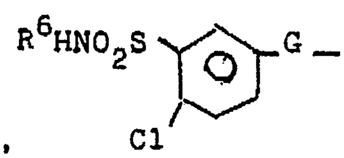

VI VII

où A est Cl ou $CF_3$ ;

D est $-(CH_2)_u-$, $-CH_2O-$, $-CH_2S-$ ;

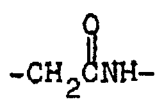

;

G est $-CONR^7(CH_2)_t-$, ou $-SO_2NR^7(CH_2)_t-$ ; t est 0 ou 1 ;

$R^1$ et $R^4$ sont indépendamment hydroxy, alcoxy ayant de 1 à 8 atomes de carbone, $K-X_r-(CH_2)_s-O-$, où K est phényle, phényle substitué, 1- ou 2-naphtyle, X est oxygène ou soufre, r est 0 ou 1 et s est 0 à 4, et où les substituants sur le phényle sont choisis dans le groupe M, où M est halogène, hydroxy, trifluorométhyle, alcoxy ayant 1 à 6 atomes de carbone, 2- et 3-furanyle, 2- et 3-thiényle et phényle (lequel groupe phényle peut être substitué par halogène, hydroxy, trifluorométhyle, alcoxy ayant 1 à 6 atomes de carbone ou alkyle ayant 1 à 6 atomes de carbone) à condition que quand s est zéro, r est zéro, $-OCH_2OCO$-alkyle où l'alkyle a 3 à 8 atomes de carbone, $-OCH_2CO$-phényle, où le phényle peut être substitué par le groupe M, 1-glycéryle,

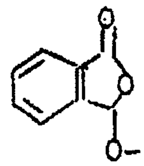

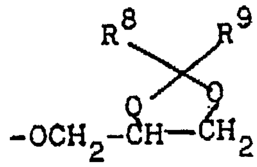

$R^2$, $R^5$, $R^6$ et $R^9$ sont hydrogène ou alkyle $C_1$-$C_6$ ;
$R^3$ est hydrogène, alkyle $C_1$-$C_6$ ou amino alkyle $C_1$-$C_6$ ;
$R^7$ est hydrogène, alkyle, $C_1$-$C_6$ où phényl-alkyle $C_1$-$c_6$ ;
$R^8$ est hydrogène, alkyle $C_1$-$C_6$, phényle, ou phényle substitué par le groupe M ;
u est 1 ou 2 ;
et ses sels acceptables en pharmacie, caractérisé en ce que

(a) un acide aminé de formule VIII est condensé avec un composé céto de formule IX en présence d'un agent réducteur dans un solvant

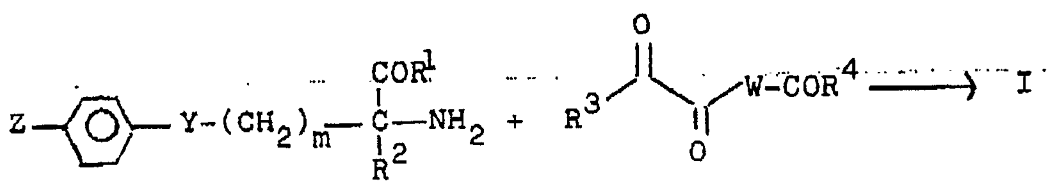

VIII IX

où Z, Y, $R^1$, $R^2$, $R^3$, $R^4$, m et W sont tels que définis ci-dessus ;

(b) pour la préparation des composés de formule I où Y est $-CH_2-$, et Z est un groupe de formule VII, on fait réagir un acide de formule XIX avec une amine de formule XX :

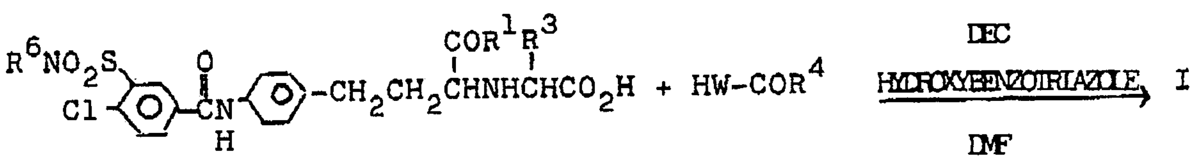


XIX XX

où $R^1$, $R^3$, $R^4$, $R^6$ et W sont tels que définis ci-dessus, et "DEC" indique 1-(3-diméthylaminopropyl)-3-éthyl carbodiimide HCl ;

(c) pour la préparation des composés de formule I où Y est $-CH_2-$, et Z est un groupe de formule VII, on fait réagir un chlorure d'acide de la formule XXVII avec un dipeptide de la formule XXVIII :

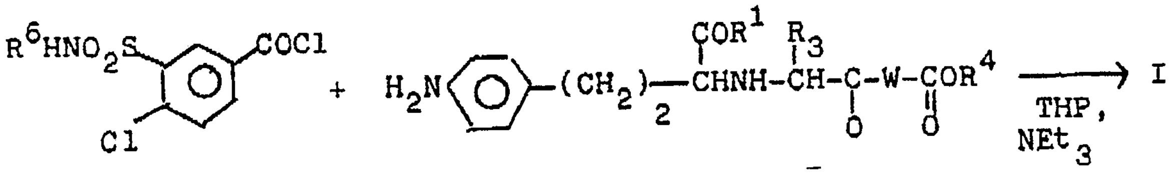


XXVII XXVIII

où $R^1$, $R^3$, $R^4$, $R^6$ et W sont tels que définis ci-dessus

avec ensuite, si on le souhaite, (i) la formation d'un sel et/ou (II) l'estérification ou la transestérification et/ou la desestérification et/ou (iii) l'isolement des isomères.

2. Méthode selon la revendication 1, où un composé est produit dans lequel W est représenté par la formule III, IV ou V.

3. Méthode selon les revendications 1 ou 2 ci-dessus, où un composé est produit où W est représenté par la formule III ou IV, où p est 0 et q est 1.

4. Méthode selon le revendication 1 où 2 ci dessous, où un composé est produit où W est représenté par la formule V, où p est 1, q est 1, et n est 0.

5. Méthode selon l'une quelconque des revendication 1-4 ci-dessus, où un composé est produit où $R^2$ et $R^5$ sont hydrogène.

6. Méthode selon l'une quelconque des revendications 1-5 ci-dessus, où un composé est produit où $R^4$ est hydroxy.

7. Méthode selon l'une quelconque des revendications 1-6 ci-dessus, où un composé est produit dans lequel $R^3$ est méthyle ou aminobutyle.

8. Méthode selon l'une quelconque des revendications 1-7 ci-dessus, où un composé est produit dans lequel A est chlore, et $R^6$ et $R^7$ sont hydrogène ou méthyle.

9. Méthode selon l'une quelconque des revendications 1-8 ci-dessus, où un composé est produit dans lequel $R^1$ est hydroxy, éthoxy, méthoxy, phénoxyéthoxy, ou pivaloyloxyméthoxy.

10. Méthode selon l'une quelconque des revendications 1-9 ci-dessus, où le composé est :
(1) acide 1-{N-[1-(S)-éthoxycarbonyl-2-[4-(3-sulfamoyl -4-chlorobenzamido)-phényl]éthyl]-(S)-alanyl}-cis ,syn -octahydro-1H -indole-2(S)-carboxylique ;
(2) acide 1-{N-[1(S)-éthoxycarbonyl-2-[4-(4-chloro-3-N -méthylsulfamoyl-benzamido)phényl] éthyl]-(S)-alanyl}-cis ,syn -octahydro-1H -indole-2(S)-carboxylique;
(3) acide 1-{N-[1(S)-carboxy-2-[4-(4-chloro-3-sulfamoylbenzamido)phényl]-éthyl]-(S)-alanyl}-cis ,syn -octahydro-1H -indole-2(S)-carboxylique ;
(4) acide 1-{N-[1-(S)-carboxy-2-[4-(4-chloro-3-N-methylsulfamoylbenzamido)-phényl]éthyl]-(S)-alanyl}-cis ,,syn -octahydro-1H -indole-2(S)-carboxylique ;
(5) acide 1-{N-[1-(S)-éthoxycarbonyl-3-[4-(4-chloro-3-sulfamoylbenzamido)-phényl]propyl]-(S)-alanyl}-cis ,syn -octahydro-1H -indole-2(S)-carboxylique ;
(6) acide 1-{N-[1-(S)-éthoxycarbonyl-3-[4-(4-chloro-3-N-méthylsulfamoyl-benzamido)phényl]propyl]-(S)-alanyl}-cis ,syn -octahydro-1H -indole-2(S)-carboxylique ;
(7) acide 1-{N-[1-(S)-éthoxycarbonyl-3-[4-(6-chloro-3,4-dihydro-1,1-dioxo-7-sulfamoyl-2H -1,2,4-benzothiadiazine-3-acetamido)-phényl]propyl]-(S)-alanyl}-cis ,syn -octahydro-1H -indole-2(S)-carboxylique ; ou un sel acceptable en pharmacie.

11. Méthode de production d'une composition pharmaceutique qui consiste à mélanger un composé de formule I tel que défini à la revendication 1, ou son sel, avec un véhicule ou excipient acceptable en pharmacie.

12. Méthode de production d'une composition pharmaceutique qui consiste à mélanger un composé de formule I ou son sel, préparé par un procédé selon l'une quelconque des revendications 1 à 10, avec un véhicule ou excipient acceptable en pharmacie.

13. Méthode de production d'une composition pharmaceutique utile pour le traitement du glaucome et/ou de l'hypertension, caractérisée par le mélange d'un composé de formule I tel que défini à la revendication 1 ou son sel, avec un véhicule ou excipient acceptable en pharmacie.

## Ansprüche

**1.** Verbindung, dargestellt durch die Formel

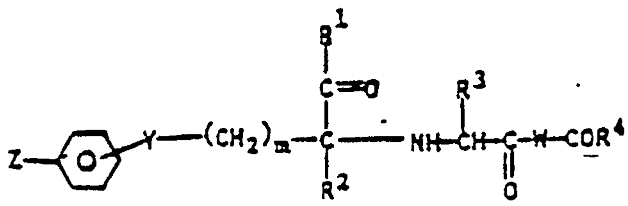

I

worin W

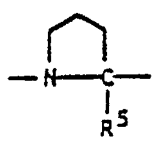 , 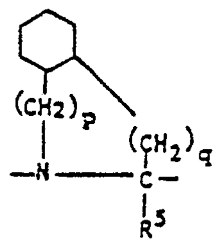 , 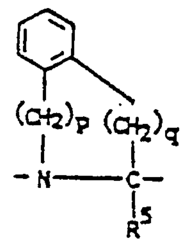 , 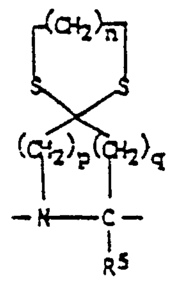 ;

II III IV V

ist,
n 0 oder 1 ist; m 0 oder 2 ist;
p und q jeweils 0, 1 oder 2 sind, vorausgesetzt, daß die Summe von p und q 1 oder 2 ist, und daß in Formel V p nicht 0 ist;
Y $-CH_2-$, $-CH_2O-$ oder $-CH_2S-$ ist, welches an die 2- oder 4-Position der Phenyl-Gruppe gebunden ist;
Z

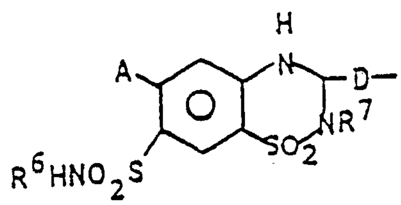 , 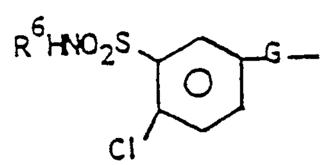

VI VII

ist, worin A Cl oder $CF_3$ ist; D $-(CH_2)_u-$, $-CH_2O$, $-CH_2S-$;

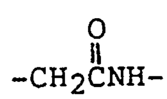

ist; G $-CONR^7(CH_2)_t-$ oder $-SO_2NR^7(CH_2)_t-$ ist; t 0 oder 1 ist;
$R^1$ und $R^4$ unabhängig Hydroxy, Alkoxy mit 1 bis 8 Kohlenstoff-Atomen, $K-X_r-(CH_2)_s-O-$, worin K Phenyl, substituiertes Phenyl, 1- oder 2-Naphthyl ist, X Sauerstoff oder Schwefel ist, r 0 oder 1 ist und s 0 bis 4 ist, und worin die Substituenten am Phenyl aus der Gruppe M ausgewählt sind, worin M Halogen, Hydroxy, Trifluormethyl, Alkoxy mit 1 bis 6 Kohlenstoff-Atomen, 2- und 3-Furanyl, 2- und 3-Thienyl und Phenyl ist (die Phenyl-Gruppe kann mit Halogen, Hydroxy, Trifluormethyl, Alkoxy mit 1 bis 6 Kohlenstoff-Atomen oder Alkyl mit 1 bis 6 Kohlenstoff-Atomen substituiert sein), vorausgesetzt, daß, wenn s Null ist, r Null ist, $-OCH_2OCO$-Alkyl, worin das Alkyl 3 bis 8 Kohlenstoff-Atome hat, $-OCH_2CO$-Phenyl, worin das Phenyl durch die Gruppe M substituiert sein kann, 1-Glyceryl,

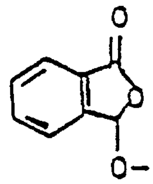

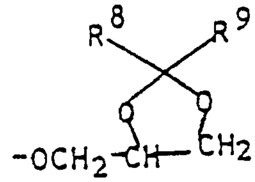

sind,
$R^2$, $R^5$, $R^6$ und $R^9$ Wasserstoff oder $C_1$-$C_6$ Alkyl sind;
$R^3$ Wasserstoff, $C_1$-$C_6$ Alkyl oder Amino-$C_1$-$C_6$-alkyl ist;
$R^7$ Wasserstoff, $C_1$-$C_6$ Alkyl oder Phenyl-$C_1$-$C_6$-alkyl ist;
$R^8$ Wasserstoff, $C_1$-$C_6$ Alkyl, Phenyl oder durch die Gruppe M substituiertes Phenyl ist;
u 1 oder 2 ist;
und deren pharmazeutisch annehmbare Salze.

**2.** Verbindung des Anspruchs 1, worin W durch die Formel III, IV oder V dargestellt wird.

**3.** Verbindung des Anspruchs 1 oder 2, worin W durch die Formel III oder IV dargestellt wird, worin p 0 ist und q 1 ist.

**4.** Verbindung eines der voranstehenden Ansprüche 1-3, worin $R^2$ und $R^5$ Wasserstoff sind.

**5.** Verbindung eines der voranstehenden Ansprüche 1-4, worin $R^4$ Hydroxy ist.

**6.** Verbindung eines der voranstehenden Ansprüche 1-5, worin $R^3$ Methyl oder Aminobutyl ist.

**7.** Verbindung eines der voranstehenden Ansprüche 1-6, worin A Chlor ist und $R^6$ und $R^7$ Wasserstoff oder Methyl sind.

**8.** Verbindung eines der voranstehenden Ansprüche 1-7, worin $R^1$ Hydroxy, Ethoxy, Methoxy, Phenoxyethoxy oder Pivaloyloxymethoxy ist.

**9.** Verbindung wie in Anspruch 1 definiert, welche
(1) 1-{N-[1-(S)-Ethoxycarbonyl-2-[4-(3-sulfamoyl-4-chlorbenzamido)-phenyl]ethyl]-(S)-alanyl}-cis ,syn -octahydro-1H-indol-2(S)-carbonsäure; ?
(2) 1-{N-[1-(S)-Ethoxycarbonyl-2-[4-(4-chlor-3-N-methylsulfamoyl-benzamido)phenyl]ethyl]-(S)-alanyl}-cis ,syn -octahydro-1H-indol-2(S)-carbonsäure;
(3) 1-{N-[1-(S)-Carboxy-2-[4-(4-chlor-3-sulfamoylbenzamido)phenyl]ethyl]-(S)-alanyl}-cis ,syn -octahydro-1H-indol-2(S)-carbonsäure;
(4) 1-{N-[1-(S)-Carboxy-2-[4-(4-chlor-3-N-methylsulfamoylbenzamido)-phenyl]ethyl]-(S)-alanyl}-cis ,syn -octahydro-1H-indol-2(S)-carbonsäure;
(5) 1-{N-[1-(S)-Ethoxycarbonyl-3-[4-(4-chlor-3-sulfamoylbenzamido)-phenyl]propyl]-(S)-alanyl)-cis ,syn -octahydro-1H-indol-2(S)-carbonsäure;
(6) 1-{N-[1-(S)-Ethoxycarbonyl-3-[4-(4-chlor-3-N-methylsulfamoyl-benzamido)phenyl]propyl]-(S)-alanyl}-cis ,syn -octahydro-1H-indol-2(S)-carbonsäure; oder
(7) 1-{N-[1-(S)-Ethoxycarbonyl-3-[4-(6-chlor-3,4-dihydro-1,1-dioxo-7-sulfamoyl-2H-1,2,4-benzothiadiazin-3-acetamido)-phenyl]propyl]-(S)-alanyl}-cis ,syn -octahydro-1H-indol-2(S)-carbonsäure ist.

**10.** Verfahren zur Herstellung einer Verbindung der Formel I, dadurch gekennzeichnet, daß
(a) eine Aminosäure der Formel VIII mit einer Keto-Verbindung der Formel IX in Gegenwart eines Reduktionsmittels in einem Lösungsmittel kondensiert wird

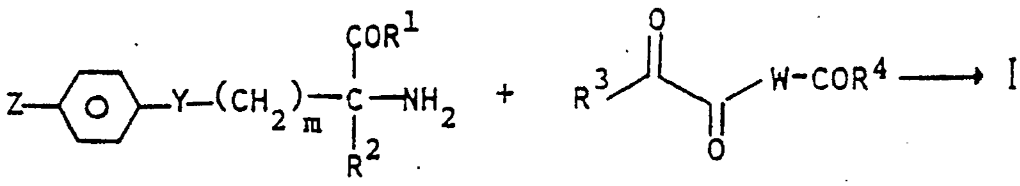

VIII IX

worin Z, Y, $R^1$, $R^2$, $R^3$, $R^4$, m und W wie voranstehend definiert sind;

(b) zur Herstellung von Verbindungen der Formel I, worin Y $-CH_2-$ ist und Z eine Gruppe der Formel VII ist, eine Säure der Formel XIX mit einem Amin der Formel XX umgesetzt wird:

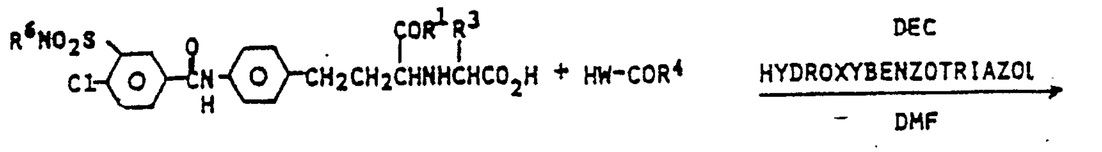


XIX XX

worin $R^1$, $R^3$, $R^4$, $R^6$ und W wie voranstehend definiert sind, und "DEC" für 1-(3-Dimethylaminopropyl)-3-ethylcarbodiimid-HCl steht;

(c) zur Herstellung von Verbindungen der Formel I, worin Y $-CH_2-$ ist und Z eine Gruppe der Formel VII ist, ein Säurechlorid der Formel XXVII mit einem Dipeptid der Formel XXVIII umgesetzt wird:

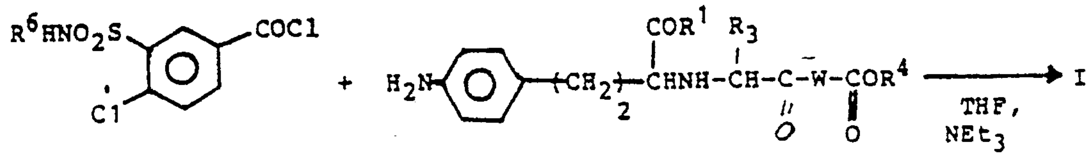


XXVII XXVIII

worin $R^1$, $R^3$, $R^4$, $R^6$ und W wie voranstehend definiert sind; falls gewünscht, gefolgt von (i) Salzbildung und/oder (ii) Veresterung oder Umesterung und/oder Entesterung und/oder (iii) Isolierung der Isomeren.

**11.** Pharmazeutische Zusammensetzung umfassend als aktiven Bestandteil wenigstens eine Verbindung wie in einem der Ansprüche 1-10 beansprucht zusammen mit einem pharmazeutischen Träger und/oder Füllstoff.

**12.** Verfahren zur Herstellung einer pharmazeutischen Zusammensetzung umfassend das Mischen einer Verbindung der Formel I mit einem pharmazeutischen annehmbaren Träger.

**13.** Verwendung einer Verbindung der Formel I zur Herstellung eines Medikaments zur Behandlung von Bluthochdruck.

**14.** Verwendung einer Verbindung der Formel I zur Herstellung eines Medikaments zur Glaukombehandlung.

Patentanspruch für folgenden Vertragsstaat: AT

**1.** Verfahren zur Herstellung einer Verbindung der Formel

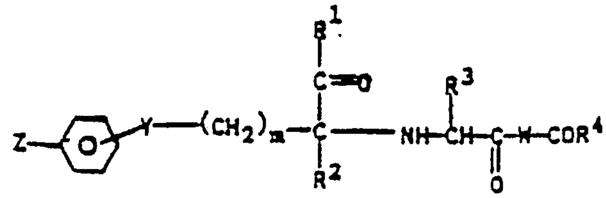

I

worin W

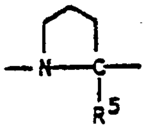 ,

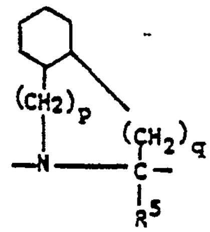 ,

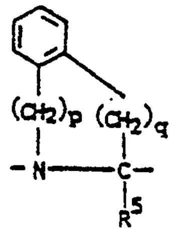 ,

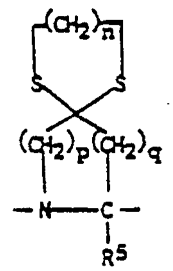 ;

II III IV V

ist,

n 0 oder 1 ist; m 0 oder 2 ist;

p und q jeweils 0, 1 oder 2 sind, vorausgesetzt, daß die Summe von p und q 1 oder 2 ist, und daß in Formel V p nicht 0 ist;

Y $-CH_2-$, $-CH_2O-$ oder $-CH_2S-$ ist, welches an die 2- oder 4-Position der Phenyl-Gruppe gebunden ist;

Z

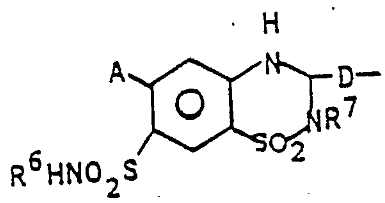 ,

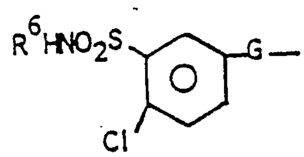

VI VII

ist, worin A Cl oder $CF_3$ ist; D $-(CH_2)u-$, $-CH_2O-$, $-CH_2S-$;

$$-CH_2\overset{\overset{O}{\|}}{C}NH-$$

ist; G $-CONR^7(CH_2)_t-$ oder $-SO_2NR^7(CH_2)_t-$ ist; t 0 oder 1 ist;

$R^1$ und $R^4$ unabhängig Hydroxy, Alkoxy mit 1 bis 8 Kohlenstoff-Atomen, $K-X_r-(CH_2)_s-O-$, worin K Phenyl, substituiertes Phenyl, 1- oder 2-Naphthyl ist, X Sauerstoff oder Schwefel ist, r 0 oder 1 ist und s 0 bis 4 ist, und worin die Substituenten am Phenyl aus der Gruppe M ausgewählt sind, worin M Halogen, Hydroxy, Trifluormethyl, Alkoxy mit 1 bis 6 Kohlenstoff-Atomen, 2- und 3-Furanyl, 2- und 3-Thienyl und Phenyl ist (die Phenyl-Gruppe kann mit Halogen, Hydroxy, Trifluormethyl, Alkoxy mit 1 bis 6 Kohlenstoff-Atomen oder Alkyl mit 1 bis 6 Kohlenstoff-Atomen substituiert sein), vorausgesetzt, daß, wenn s Null ist, r Null ist, $-OCH_2OCO-$Alkyl, worin das Alkyl 3 bis 8 Kohlenstoff-Atome hat, $-OCH_2CO-$Phenyl, worin das Phenyl durch die Gruppe M substituiert sein kann, 1-Glyceryl,

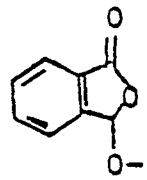

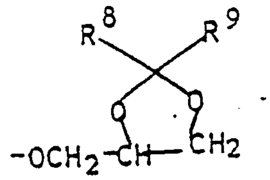

sind,
$R^2$, $R^5$, $R^6$ und $R^9$ Wasserstoff oder $C_1$-$C_6$ Alkyl sind;
$R^3$ Wasserstoff, $C_1$-$C_6$ Alkyl oder Amino-$C_1$-$C_6$-alkyl ist;
$R^7$ Wasserstoff, $C_1$-$C_6$ Alkyl oder Phenyl-$C_1$-$C_6$-alkyl ist;
$R^8$ Wasserstoff, $C_1$-$C_6$ Alkyl, Phenyl oder durch die Gruppe M substituiertes Phenyl ist;
u 1 oder 2 ist;
und deren pharmazeutisch annehmbarer Salze, dadurch gekennzeichnet, daß

(a) eine Aminosäure der Formel VIII mit einer Keto-Verbindung der Formel IX in Gegenwart eines Reduktionsmittels in einem Lösungsmittel kondensiert wird

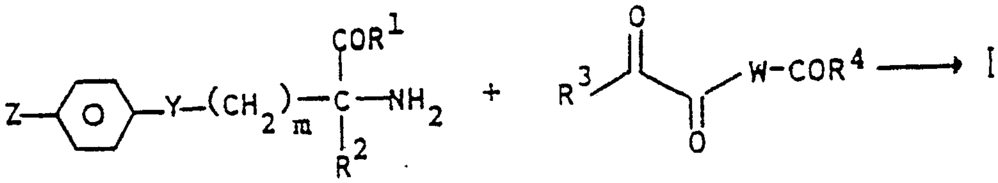

VIII IX

worin Z, Y, $R^1$, $R^2$, $R^3$, $R^4$, m und W wie voranstehend definiert sind;

(b) zur Herstellung von Verbindungen der Formel I, worin Y $-CH_2-$ ist und Z eine Gruppe der Formel VII ist, eine Säure der Formel XIX mit einem Amin der Formel XX umgesetzt wird:

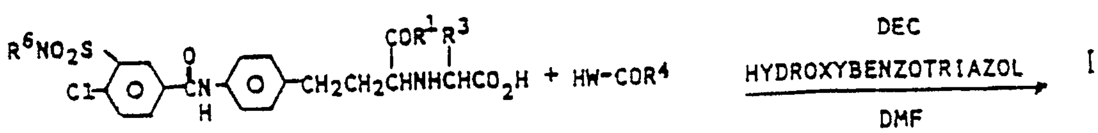


XIX XX

worin $R^1$, $R^3$, $R^4$, $R^6$ und W wie voranstehend definiert sind, und "DEC" für 1-(3-Dimethylaminopropyl)-3-ethylcarbodiimid-HCl steht;

(c) zur Herstellung von Verbindungen der Formel I, worin Y $-CH_2-$ ist und Z eine Gruppe der Formel VII ist, ein Säurechlorid der Formel XXVII mit einem Dipeptid der Formel XXVIII umgesetzt wird:

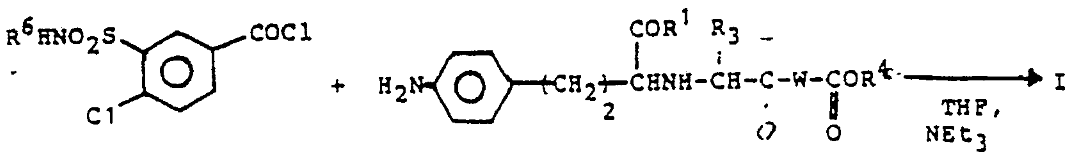


XXVII XXVIII

worin $R^1$, $R^3$, $R^4$, $R^6$ und W wie voranstehend definiert sind; falls gewünscht, gefolgt von (i) Salzbildung und/oder (ii) Veresterung oder Umesterung und/oder Entesterung und/oder (iii) Isolierung der Isomeren.

2. Verfahren gemäß Anspruch 1, wobei eine Verbindung hergestellt wird, worin W durch die Formel III, IV

oder V dargestellt wird.

3. Verfahren gemäß den voranstehenden Ansprüchen 1 oder 2, wobei eine Verbindung hergestellt wird, worin W durch die Formel III oder IV dargestellt wird, worin p 0 ist und q 1 ist.

4. Verfahren gemäß den voranstehenden Ansprüchen 1 oder 2, wobei eine Verbindung hergestellt wird, worin W durch die Formel V dargestellt wird, worin p 1 ist, q 1 ist und n 0 ist.

5. Verfahren gemäß einem der voranstehenden Ansprüchen 1-4, wobei eine Verbindung hergestellt wird, worin $R^2$ und $R^5$ Wasserstoff sind.

6. Verfahren gemäß einem der voranstehenden Ansprüche 1-5, wobei eine Verbindung hergestellt wird, worin $R^4$ Hydroxy ist.

7. Verfahren gemäß einem der voranstehenden Ansprüche 1-6, wobei eine Verbindung hergestellt wird, worin $R^3$ Methyl oder Aminobutyl ist.

8. Verfahren gemäß einem der voranstehenden Ansprüche 1-7, wobei eine Verbindung hergestellt wird, worin A Chlor ist und $R^6$ und $R^7$ Wasserstoff oder Methyl sind.

9. Verfahren gemäß einem der voranstehenden Ansprüche 1-8, wobei eine Verbindung hergestellt wird, worin $R^1$ Hydroxy, Ethoxy, Methoxy, Phenoxyethoxy oder Pivaloyloxymethoxy ist.

10. Verfahren gemäß einem der voranstehenden Ansprüche 1-9, in welchen die Verbindung
1-{N-[1-(S)-Ethoxycarbonyl-2-[4-(3-sulfamoyl-4-chlorbenzamido)-phenyl]ethyl]-(S)-alanyl}-cis ,syn -octahydro-1H-indol-2(S)-carbonsäure;
1-{N-[1-(S)-Ethoxycarbonyl-2-[4-(4-chlor-3-N-methylsulfamoyl-benzamido)phenyl]ethyl]-(S)-alanyl]-cis ,syn -octahydro-1H-indol-2(S)-carbonsäure;
1-{N-[1(S)-Carboxy-2-[4-(4-chlor-3-sulfamoyl-benzamido)phenyl]ethyl]-(S)-alanyl}-cis ,syn -octahydro-1H-indol-2(S)-carbonsäure;
1-{N-[1(S)-Carboxy-2-[4-(4-chlor-3-N-methylsulfamoyl-benzamido)-phenyl]ethyl]-(S)-alanyl}-cis ,syn -octahydro-1H-indol-2(S)-carbonsäure;
1-{N-[1-(S)-Ethoxycarbonyl-3-[4-(4-chlor-3-sulfamoyl-benzamido)-phenyl]propyl]-(S)-alanyl}-cis ,syn -octahydro-1H-indol-2(S)-carbonsäure;
1-{N-[1-(S)-Ethoxycarbonyl-3-[4-(4-chlor-3-N-methylsulfamoyl-benzamido)phenyl]propyl]-(S)-alanyl}-cis ,syn -octahydro-1H-indol-2(S)-carbonsäure;
1-{N-[1-(S)-Ethoxycarbonyl-3-[4-(6-chlor-3,4-dihydro-1,1-dioxo-7-sulfamoyl-2H-1,2,4-benzothiadiazin-3-acetamido)-phenyl]propyl]-(S)-alanyl}-cis ,syn -octahydro-1H-indol-2(S)-carbonsäure oder ein pharmazeutisch annehmbares Salz ist.

11. Verfahren zur Herstellung einer pharmazeutischen Zusammensetzung, welches das Mischen einer wie in Anspruch 1 definierten Verbindung der Formel I mit einem pharmazeutischen annehmbaren Träger oder Füllstoff umfaßt.

12. Verfahren zur Herstellung einer pharmazeutischen Zusammensetzung, welches das Mischen einer nach einem der in einem der Ansprüche 1 bis 10 beanspruchten Verfahren hergestellten Verbindung der Formel I oder deren Salz mit einem pharmazeutischen annehmbaren Träger oder Füllstoff umfaßt.

13. Verfahren zur Herstellung einer zur Behandlung von Glaukom und/oder Bluthochdruck nützlichen pharmazeutischen Zusammensetzung, gekennzeichnet durch Vermischen einer wie in Anspruch 1 definierten Verbindung der Formel I oder deren Salz mit einem pharmazeutisch annehmbaren Träger oder Füllstoff.